(19) **Europäisches Patentamt / European Patent Office / Office européen des brevets**

(11) **EP 3 872 173 A1**

(12) # EUROPEAN PATENT APPLICATION

(43) Date of publication:
**01.09.2021 Bulletin 2021/35**

(51) Int Cl.:
***C12N 9/42*** *(2006.01)*    ***C08L 1/02*** *(2006.01)*

(21) Application number: **20382141.8**

(22) Date of filing: **28.02.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **CIC nanoGUNE - Asociación Centro de Investigación Cooperativa en Nanociencias**
  **20018 San Sebastian, Guipuzcoa (ES)**
- **Universidad del País Vasco/Euskal Herriko Unibertsitatea**
  **48940 Leioa, Bikaia (ES)**

(72) Inventors:
- **PÉREZ JIMÉNEZ, Raúl**
  **E-20018 San Sebastián, Guipúzcoa (ES)**
- **ALONSO LEMA, Borja**
  **E-20018 San Sebastián, Guipúzcoa (ES)**
- **ECEIZA MENDIGUREN, María Aranzazu**
  **E-48940 Leioa, Vizcaya (ES)**

(74) Representative: **ABG Intellectual Property Law, S.L.**
**Avenida de Burgos, 16D**
**Edificio Euromor**
**28036 Madrid (ES)**

(54) **METHOD FOR PRODUCING CRYSTALLINE NANOCELLULOSE**

(57)    The invention relates to a method for producing crystalline nanocellulose comprising contacting a substrate comprising cellulose with an enzyme comprising an endoglucanase catalytic domain under suitable conditions for hydrolyzing cellulose and under a pH greater than 5, wherein the endoglucanase catalytic domain comprises a sequence selected from the group consisting of SEQ ID NO: 1, SEQ ID NO: 2, SEQ ID NO: 3 and a functionally equivalent variant thereof that substantially maintains or improves its endoglucanase catalytic activity. The invention also relates to crystalline nanocellulose obtained by this method.

EP 3 872 173 A1

**Description**

**FIELD OF THE INVENTION**

The present invention falls within the field of methods for the enzymatic production of crystalline nanocellulose.

**BACKGROUND OF THE INVENTION**

Nanocellulose is a novel biomaterial that has been extensively studied due to its extraordinary features such as biocompatibility, renewability, sustainability, its nanometer size, large aspect ratio, flexibility, electrical, thermal and mechanical features. Numerous applications have been proposed for nanocellulose, ranging from biomedicine to electronics. These applications are determined by the type of nanocellulose used which depends on size, shape, crystallinity, and source. Cellulose has a hierarchical organization from where different types of nanocellulose can be derived. Cellulose nanocrystals (CNCs) are small crystalline particles with lengths that vary from 100 to 500 nm depending on their source and isolation treatment.

Cellulose nanofibers (CNFs) are longer thin fibers with lengths of up to several micrometers, although CNCs have higher mechanical stability in comparison with CNFs due to their crystalline structure. CNCs have high surface areas and tensile strength that can be compared with other materials such as Kevlar or carbon nanotubes.

Currently, there are different methods for nanocellulose production: mechanical, chemical, enzymatic treatments or a combination of them. Mechanical treatments consist on high-pressure homogenization cycles that generally generate CNFs (Iwamoto, S. et al., Appl. Phys. A 220 89; 461-466). This method needs numerous repeating steps and chemicals or enzymatic treatments (Henriksson, M. et al., Eur. Polym. J. 2007, 43: 3434-3441) are usually needed in order to reduce energy consumption. Chemical treatments are the main procedure for CNCs isolation. Sulfuric acid hydrolysis is the common reagent that swells cellulose amorphous regions keeping the crystalline part. During this process, sulfate groups attach to the CNCs surface by an esterification reaction. Sulfates stabilize nanocrystals in water suspension but have other effects in the CNCs physicochemical properties such as a decrease in crystallinity and thermal stability. Moreover, this method produces large amounts of residual wastewaters after the neutralization and dialysis steps, making it very pollutant. Enzymatic treatment is the preferred path for biomass hydrolysis and it also represents an ideal alternative for nanocellulose production. Although, enzymatic hydrolysis has been used in combination with mechanical and chemical treatments to improve yields (Zhu, J.Y. et al., Green Chem. 2011, 13: 1339-1344; Satyarmurthy, P. et al., Carbohydr. Polym. 2011, 83: 122-129) nanocellulose isolation using only enzymes remains largely unexplored, with only one notable example utilizing a cellulase enzyme complex from *Caldicellulosiruptor bescii* (Yarbrough, J. M. et al., Acs Nano 2017, 11: 3101-3109). Commercial fungal cellulases cocktail is optimized for sugar production and is not suitable for nanocellulose obtention. Nevertheless, typical fungal enzymes used in biomass treatment have shown limitations in the production of CNC (Yarbrough, J. M. et al., Acs Nano 2017, 11: 3101-3109). Therefore, the development of highly efficient enzymes suited for nanocellulose production as well as the optimization of the obtention process are still needed for the enzymatic production of nanocellulose

**SUMMARY OF THE INVENTION**

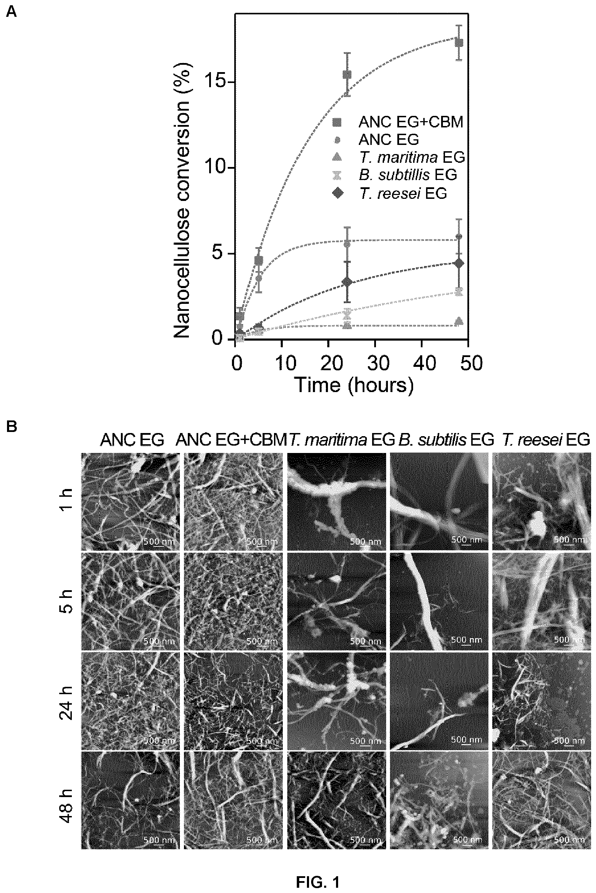

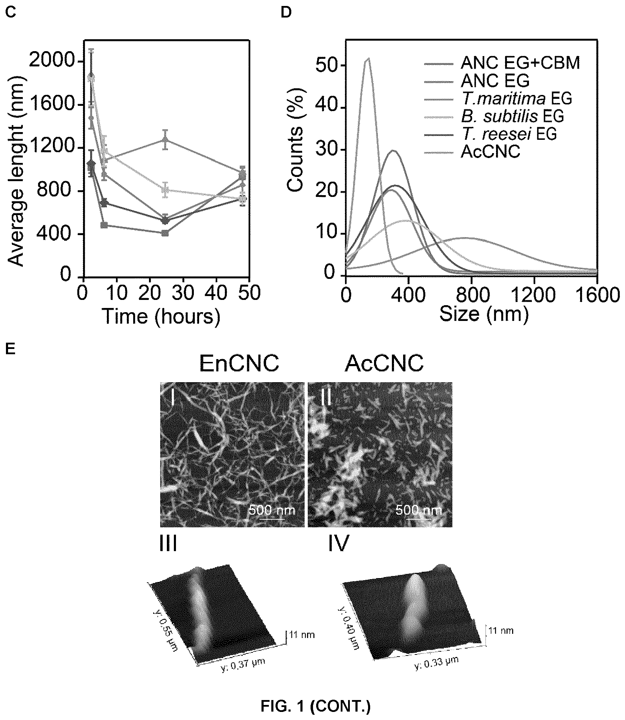

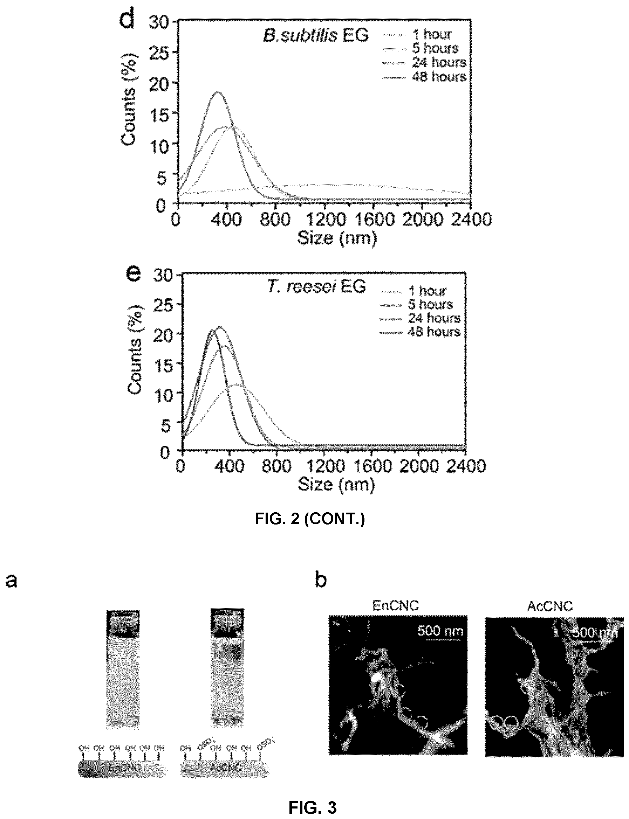

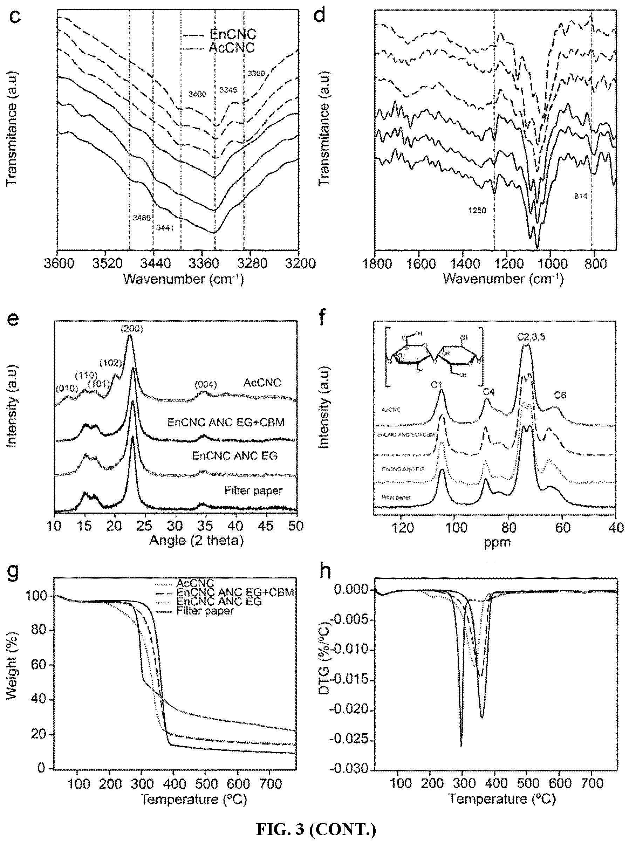

The authors of the present invention have developed a method for the sustainable production of pure, size-controlled and highly crystalline nanocellulose using an ancestral endoglucanase reconstructed from bacteria species. In particular, the inventors have shown that the ancestral endoglucanase is capable of producing both CNFs and CNCs from filter paper with higher yields than those of other modern EGs from *T. maritima, T. reesei and B. subtilis* (Fig. 1). Using a battery of techniques to provide a full and detailed characterization of this enzymatically produced nanocrystals (EnCNC), the inventors have demonstrated that these EnCNC maintain native cellulose structure with higher crystallinity and thermal stability in comparison to commercial nanocrystal isolated by sulfuric acid hydrolysis (AcCNC) (Fig. 3).

Thus, in an aspect, the invention relates to a method for producing crystalline nanocellulose comprising contacting a substrate comprising cellulose with an enzyme comprising an endoglucanase catalytic domain under suitable conditions for hydrolyzing cellulose and under a pH greater than 5, wherein the endoglucanase catalytic domain comprises a sequence selected from the sequence selected from the group consisting of SEQ ID NO: 1, SEQ ID NO: 2, SEQ ID NO: 3 and a functionally equivalent variant thereof that substantially maintains or improves its endoglucanase catalytic activity.

In another aspect, the invention relates to crystalline nanocellulose obtainable or obtained by the method of the above aspect.

## BRIEF DESCRIPTION OF THE FIGURES

**Figure 1. Nanocellulose yields from enzymatic hydrolysis, average particle length and AFM images.** (a) Nanocellulose conversion from filter paper using ANC EG, ANC EG+CBM and EG from *T. maritima, B. subtilis* and *T. reesei* at 50°C at different times. ANC EG+CBM had around three times the activity of ANC EG and even bigger differences respect to modern enzymes. The average yield and S.D. were calculated from three independent experiments. (b) Average size of nanocellulose from filter paper using ANC EG, ANC EG+CBM and EG from *T. maritima, B. subtillis* and *T. reesei* at 50°C at different times. The smaller length was measured after 24 hours ANC EG+CBM hydrolysis. The average yield and S.D. were calculated from three independent experiments. (c) Comparison of size distribution of nanocellulose produced with enzymatic hydrolysis and AcCNC. Acid hydrolysis produces more homogeneous suspension of nanocellulose than the enzymatic treatment, and between EGs, ANC EG+CBM produced the more similar population. The distribution was calculated with the length of 100 particles from each condition. (d) AFM images of nanoparticles produced by EG hydrolysis at different time points (5 $\mu$m x 5 $\mu$m). The images show the nanoparticles that the EGs produced at 1, 5, 24, and 48 hours using filter paper as a substrate. The enzymatic hydrolysis produced both CNFs and CNCs depending on the enzyme and the time of the process. (e) AFM images of nanoparticles produced with ANC EG+CBM hydrolysis and AcCNC (3 $\mu$m x 3 $\mu$m). Height images (I) of nanocellulose produced by ancestral endoglucanase with CBM at 24 hours hydrolysis using filter paper as substrate and (II) of nanocellulose produced by sulfuric acid hydrolysis purchased from Maine University. (III) A 3D image of a single EnCNC, showing the needle-like shape and (IV) a 3D image from the AcCNC sample with ribbon-like shape.

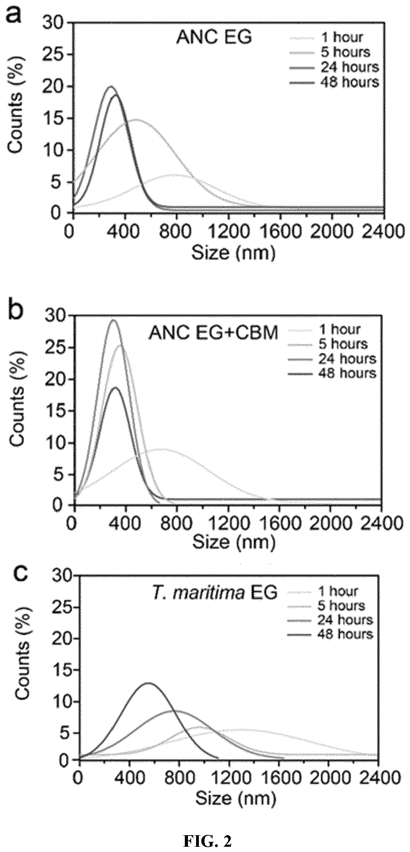

**Figure 2.** Size distribution of nanocellulose particles produced with the (a) ANC EG, (b) ANC EG+CBM, (c) *T. maritima* EG (d) *B. subtillis* EG (e) *T. reesei* EG using filter paper as a substrate at different time points. The figure shows the smaller and more homogeneous size of the nanoparticle's population using ANC EG+CBM. The distribution was calculated with the length of 100 particles from each condition.

**Figure 3.** Nanocellulose physicochemical characterization. (a) Aqueous suspension of EnCNCs and AcCNCs. The high charged surface of AcCNC made them easier to disperse in water while the EnCNC was less stable due to CNC agglomeration by hydrogen bonding. Illustration of free -OH groups in EnCNC surface and the random - $OSO_3^-$ groups anchored by the sulfuric acid treatment in the AcCNC surface. (b) Topography images from EnCNC and AcCNC samples used for nano-FTIR measurement. Circles mark the analyzed nanocrystals. (c) Spectra from EnCNC and AcCNC nanocrystals selected in two different regions, 3600 to 3200 cm-1 and (d), 1800 to 750 cm-1. Spectra form EnCNC and AcCNC showed different peaks due to its different polymorph structure and AcCNC sulfate content. (e) X-ray diffraction analysis of the filter paper, EnCNCs produced by 24 hours hydrolysis of ANC EG and ANC EG+CBM and AcCNCs. Figure shows the diffractograms of cellulose I in the filter paper and EnCNCs. The different planes are indicated. (f) CP/MAS $^{13}$C NMR spectra of the filter paper, EnCNCs produced by 24 hours hydrolysis of ANC EG and ANC EG+CBM and AcCNC. Different carbons are indicated and correlate with both crystalline and amorphous components of cellulose. (g) Thermogravimetric analysis curves of filter paper, EnCNCs produced by 24 hours hydrolysis of ANC EG and ANC EG+CBM and AcCNCs. (h) Weight loss curves and derivative from TGA curves. The curves showed higher thermal stability of EnCNC compared to AcCNC.

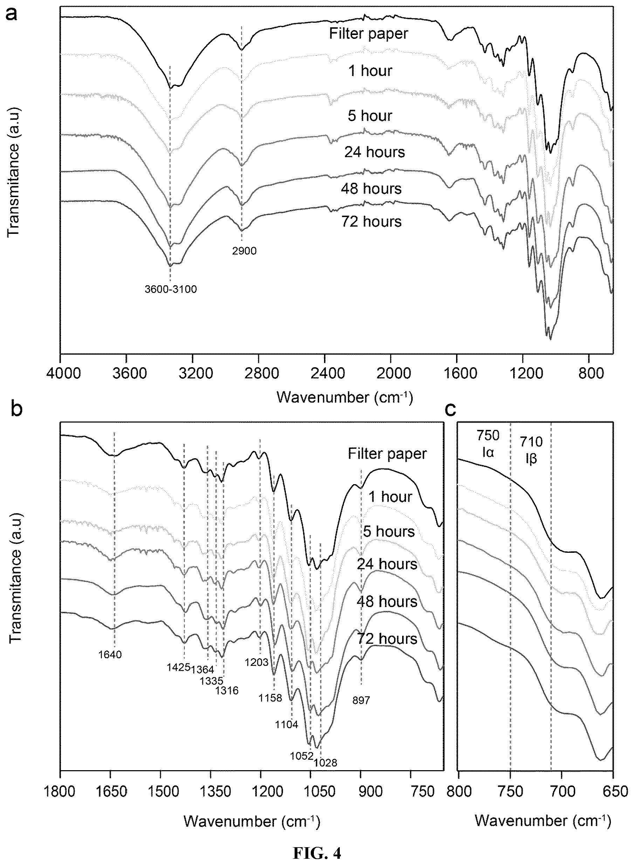

**Figure 4.** FTIR spectra of nanocellulose produced by ANC EG hydrolysis of filter paper at 1, 5, 24, 48 and 72 hours. (a) Spectra from 4000 to 650 cm-1, (b) an amplification from 1800 to 650 cm-1, and (c) from 800 to 650 cm-1. In the figure it can be observed that ANC EG did not produce any chemical change in the filter paper.

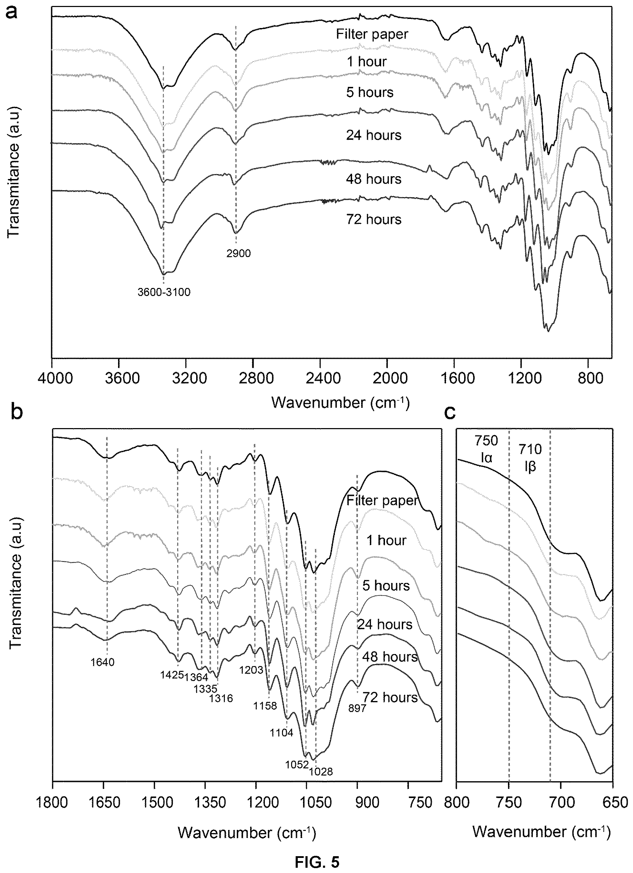

**Figure 5.** FTIR spectra of nanocellulose produced by ANC EG+CBM hydrolysis of filter paper at 1, 5, 24, 48 and 72 hours. (a) Spectra from 4000 to 750 cm-1, (b) an amplification from 1800 to 750 cm-1 and (c) from 800 to 650 cm-1. In the figure it can be observed that ANC EG+CBM did not change the chemical structure of filter paper as the ANC EG.

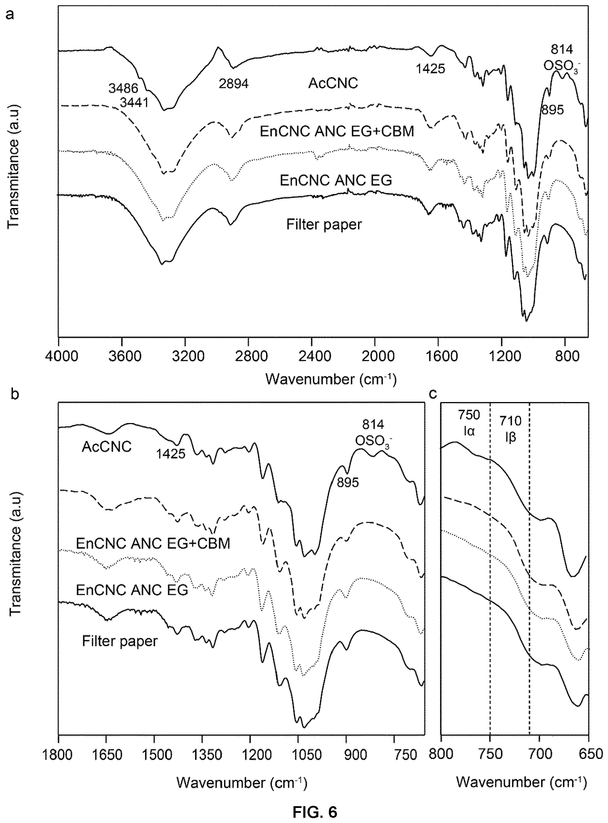

**Figure 6.** Comparison of FTIR spectra of filter paper, sulfuric acid hydrolysis produced nanocellulose and nanocellulose produced by ANC EG and ANC EG+CBM hydrolysis at 24 hours. (a) Spectra from 4000 to 650 cm-1, (b) an amplification from 1800 to 650 cm-1 and (c) from 800 to 650 cm-1. In the figure it can be observed the chemical structure changes between enzymatic produced nanocellulose and acid produced one.

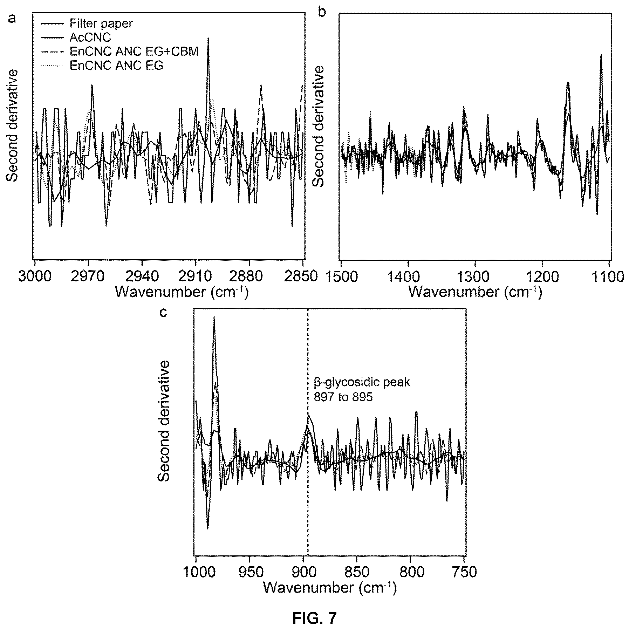

**Figure 7.** Second derivative FTIR spectra of filter paper, EnCNC from hydrolysis with both ANC EGs and AcCNC, (a) between 3000-2850 cm-1, (b) between 1500-1100 cm-1 and (c) from 1000-750 cm-1.

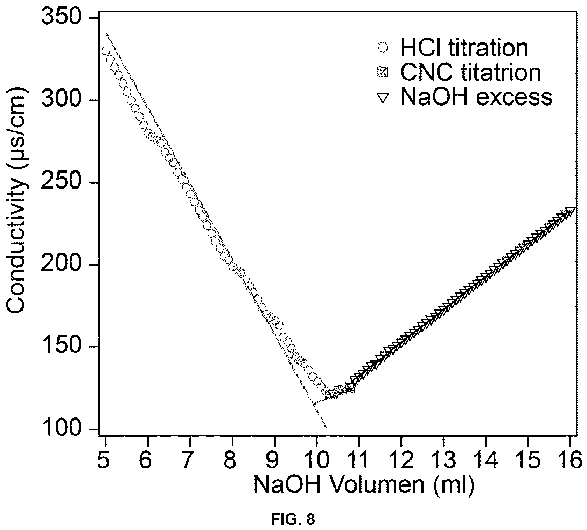

**Figure 8.** Conductometric titration curve of cellulose nanocrystals produced by sulfuric acid hydrolysis. The characterization was performed to corroborate the sulfur content given by the manufacturer, the %S measured was 0.95%±0.04 and the manufacturer data was 0.94%. The average and S.D. were calculated from two independent experiments.

**DETAILED DESCRIPTION OF THE INVENTION**

In a first aspect, the invention relates to a method for producing crystalline nanocellulose comprising contacting a substrate comprising cellulose with an enzyme comprising an endoglucanase catalytic domain under suitable conditions for hydrolyzing cellulose and under a pH greater than 5, wherein the endoglucanase catalytic domain comprises a sequence selected from the group consisting of SEQ ID NO: 1, SEQ ID NO: 2, SEQ ID NO: 3 and a functionally equivalent variant thereof that substantially maintains or improves its endoglucanase catalytic activity.

The term "crystalline nanocellulose" or "cellulose nanocrystals" or "CNC" or "nanowhiskers", as used herein, refers to a still rod-like nanoparticles consisting of cellulose chain segments with very high crystallinity. CNC have normally an average diameter between 2-30 nm and a length of 10 nm to 2 $\mu$m, depending on the cellulose source and the isolation treatment, as opposed to cellulose nanofibers (CNF), which are long thin fibers with a length of several micrometers and diameters of nanometers, and include both amorphous and crystalline domains. Another difference between CNC and CNF is the aspect ratio (length/diameter). It is generally accepted that CNF have an aspect ratio over 100, while the aspect ratio of CNC is generally lower.

The method of the invention comprises contacting a substrate comprising cellulose with an enzyme comprising an endoglucanase catalytic domain comprising a sequence selected from the group consisting of SEQ ID NO: 1, SEQ ID NO: 2, SEQ ID NO: 3 and a functionally equivalent variant thereof that substantially maintains or improves its endoglucanase catalytic activity.

The term "cellulose" as used herein, refers to an organic compound with CAS number 9004-34-6, a polysaccharide consisting of a linear chain of several hundred to many thousands of $\beta(1{\rightarrow}4)$ linked D-glucose units. Cellulose is a semi-crystalline polymer comprising crystalline and amorphous regions. Crystalline domains are very packed together and are very difficult to degrade by either chemical or enzymatic treatment. The amorphous regions are easier to degrade. The proportion between the amorphous and the crystalline regions depends on the cellulose source and on the treatment used to extract the fibers. There are six polymorphs described in the literature: cellulose I, II, III$_I$, III$_{II}$, IV$_I$ and IV$_{II}$. The native and most abundant form of cellulose is type I, and all polymorphs can be produced from it with different physicochemical treatments. Cellulose I has the chains of the polymer in a parallel organization and it can also be organized in two polymorphs, cellulose I$\alpha$ and I$\beta$. These two type I polymorphs can be found together, and the ratio between them vary with the cellulose source. Cellulose I$\alpha$ is abundant in algae and bacterial cellulose and cellulose I$\beta$ is more present in higher plants and tunicates. Cellulose II is the second most abundant cellulose form; in this polymorph, cellulose chains have an antiparallel organization. This distribution permits that hydrogen bonds happen between the neighbor's hydroxyl groups and improved the interlayer attraction forces, but there are less secondary hydrogen bonds. These interactions made that cellulose II has added stability and produce the irreversibility to convert cellulose II into I. Also, cellulose II is more thermostable and weaker mechanically than cellulose I because of its different chain organization. Cellulose II can be prepared from cellulose I by mercerization (alkaline treatment) or regeneration. From cellulose I and II the other polymorphs can be produced. Cellulose III is made from cellulose I or cellulose II by ammonia treatment that penetrates and degrades the cellulose crystal structure. Cellulose IV is produced from cellulose III by glycerol and heat treatment at 260 °C. Cellulose III can be transformed into their previous polymorphs by alkaline treatment.

In a particular embodiment, the substrate comprising cellulose comprises amorphous and crystalline regions. In a particular embodiment, the substrate comprises type I cellulose.

The term "substrate comprising cellulose", as use herein, refers to any material comprising cellulose, preferably type I cellulose. Illustrative non-limitative examples of said substrates are lignocellulose biomass (composed mainly of cellulose, hemicellulose and lignin), corn stover, *Panicum virgatum* (switchgrass), Miscanthus grass species, wood chips and the byproducts of lawn and tree maintenance. Lignocellulosic biomass can be grouped into four main categories: (1) agricultural residues (including corn stover and sugarcane bagasse), (2) dedicated energy crops, (3) wood residues (including sawmill and paper mill discards), and (4) municipal paper waste. Illustrative lignocellulosic biomass sources include, but are not limited to grasses, rice hulls, bagasse, jute, hemp, flax, bamboo, sisal, abaca, straw, corn cobs, corn stover, alfalfa, hay, coconut hair, seaweed and algae.

In a particular embodiment, the substrate comprising cellulose comprises or consists on pure cellulose. The term "pure cellulose", as used herein, refers to cellulose that has generally been separated from other plant natural products, such as hemicellulose and/or lignin and typically has a purity of 90% w/w or more, e.g. 95% w/w or more. Examples of pure cellulose substrates are filter paper or cellulose threads. In a more particular embodiment, the substrate comprising or consisting of pure cellulose is filter paper.

In a particular embodiment, the substrate comprising cellulose is a lignocellulosic substrate. The term "lignocellulosic substrate", as used herein, refers to a material, usually derived from plant biomass, which comprises cellulose, hemicellulose and lignin. The lignocellulosic material can be derived from a single material or a combination of materials and/or can be non-modified and/or modified. Lignocellulosic material can be transgenic (i.e., genetically modified). Lignocellulose is generally found, for example, in the fibers, pulp, stems, leaves, hulls, canes, husks, and/or cobs of plants or fibers, leaves, branches, bark, and/or wood of trees and/or bushes. Examples of lignocellulosic materials include, but are not limited to, agricultural biomass, e.g., farming and/or forestry material and/or residues, branches, bushes, canes, forests, grains, grasses, short rotation woody crops, herbaceous crops, and/or leaves; oil palm fiber waste such as empty fruit bunch and palm trunk; energy crops, e.g., corn, millet, and/or soybeans; energy crop residues; paper mill residues; sawmill residues; municipal paper waste; orchard prunings; Willow coppice and Mallee coppice; wood waste; wood chip, logging waste; forest thinning; short-rotation woody crops; bagasse, such as sugar cane bagasse and/or sorghum bagasse, duckweed; wheat straw; oat straw; rice straw; barley straw; rye straw; flax straw; soy hulls; rice hulls; rice straw; tobacco; corn gluten feed; oat hulls; corn kernel; fiber from kernels; corn stover; corn stalks; com cobs; corn husks; canola; miscanthus; energy cane; prairie grass; gamagrass; foxtail; sugar beet pulp; citrus fruit pulp; seed hulls; lawn clippings; cotton, seaweed; trees; shrubs; wheat; wheat straw; products and/or byproducts from wet or dry milling of grains; yard waste; plant and/or tree waste products; herbaceous material and/or crops; forests; fruits; flowers; needles; logs; roots; saplings; shrubs; switch grasses; vegetables; fruit peels; vines; wheat midlings; oat hulls; hard and soft woods; or any combination thereof. In another embodiment, the lignocellulosic material may be the product obtained by a processor selected from the group consisting of a dry grind ethanol production facility, a paper pulping facility, a tree harvesting operation, a sugar cane factory, or any combination thereof.

In a particular embodiment, the lignocellulosic material is paper pulp.

The term "paper pulp" means any pulp which can be used for the production of a paper material. For example, the pulp can be supplied as a virgin pulp, or can be derived from a recycled source. The pulp may be a wood pulp, a non-wood pulp or a pulp made from waste paper. A wood pulp may be made from softwood such as pine, redwood, fir, spruce, cedar and hemlock or from hardwood such as maple, alder, birch, hickory, beech, aspen, acacia and eucalyptus. A non-wood pulp may be made, e.g., from flax, hemp, bagasse, bamboo, cotton or kenaf. A waste paper pulp may be made by re-pulping waste paper such as newspaper, mixed office waste, computer print-out, white ledger, magazines, milk cartons, paper cups etc. The wood pulp may be mechanical pulp (such as ground wood pulp, GP), chemical pulp (such as Kraft pulp or sulfite pulp), semichemical pulp (SCP), thermomechanical pulp (TMP), chemithermomechanical pulp (CTMP), or bleached chemithermomechanical pulp (BCTMP).

Mechanical pulp is manufactured by the grinding and refining methods, wherein the raw material is subjected to periodical pressure impulses. TMP is thermomechanical pulp, GW is groundwood pulp, PGW is pressurized groundwood pulp, RMP is refiner mechanical pulp; PRMP is pressurized refiner mechanical pulp and CTMP is chemithermimechanical pulp.

Chemical pulp is manufactured by alkaline cooking whereby most of the lignin and hemicellulose components are removed. In Kraft pulping or sulphate cooking sodium sulphide or sodium hydroxide are used as principal cooking chemicals.

In a particular embodiment, the lignocellulosic material is unbleached kraft pulp (KAPPA). KAPPA is a pulp rich in cellulose (about 70%) but also comprising hemicellulose (about 10%), lignin (about 12%) and other components as described by Da Silva, T.A. et al., BioResources 2007, 2(4): 616-629. In another particular embodiment, the lignocellulosic material is bleached kraft pulp (BKP). BKP comprises more cellulose (about 80%), hemicellulose (12%) and a small lignin residue (about 1%) as described by Hu, J. et al., Sci. Rep. 2018, 8(1): 3195.

The term "enzyme comprising an endoglucanase catalytic domain", or "endoglucanase" as used herein, refers to an enzyme that randomly cleaves (1-4)-β-D-glucosidic links in cellulose, lichenin and cereal β-D-glucans, thereby creating new chain ends. Endoglucanases also hydrolyse 1,4-linkages in β-D-glucans also containing 1,3-linkages. It has been classified by the Nomenclature Committee of the International Union of Biochemistry and Molecular Biology as EC 3.2.1.4. Endoglucanases derived from bacteria have a catalytic domain and a carbohydrate binding module attached by a linker or linking domain. However, in the present invention, the term "endoglucanase" is not restricted to enzymes comprising a carbohydrate binding motive, but encompasses any enzyme comprising an endoglucanase catalytic domain, therefore having endoglucanase activity, as previously defined

The term "endoglucanase catalytic domain", as used herein, refers to a domain of an enzyme being responsible of its catalytic function, particularly of its endoglucanase function. It contains the active site, a set of amino acids with a special spatial arrangement that permits interaction with the substrate to effect the reaction.

In the method of the invention, the endoglucanase catalytic domain comprises or consists on a sequence selected from the group consisting of SEQ ID NO: 1, 2, 3 and a functionally equivalent variant thereof.

In a particular embodiment, the endoglucanase catalytic domain comprises or consists on the sequence of SEQ ID NO: 1.

In a particular embodiment, the endoglucanase catalytic domain comprises or consists on a functionally equivalent variant of the sequences of SEQ ID NO: 1, 2, or 3, preferably of SEQ ID NO: 1.

The term "functionally equivalent variant" as used herein is understood to mean all those proteins derived from a sequence by modification, insertion and/or deletion or one or more amino acids, whenever the function is substantially maintained, particularly in the case of a functionally equivalent variant of an endoglucanase catalytic domain refers to maintaining the endoglucanase catalytic activity.

A functionally equivalent variant of the catalytic domain of sequence SEQ ID NO: 1, 2 or 3 can be an amino acid sequence derived from SEQ ID NO: 1, 2 or 3 comprising the addition, substitution or modification of one or more amino acid residues. By way of illustration, functionally equivalent variants of the catalytic domain of sequence SEQ ID NO: 1, 2 or 3 include sequences comprising the addition of 1 amino acid, 2 amino acids, 3 amino acids, 4 amino acids, 5 amino acids, 10 amino acids, 15 amino acids, 20 amino acids, 25 amino acids, 30 amino acids, 35 amino acids, 40 amino acids, 45 amino acids, 50 amino acids, 60 amino acids, 70 amino acids, 80 amino acids, 90 amino acids, 100 amino acids, 150 amino acids, 200 amino acids, at least 500 amino acids, at least 1000 amino acids or more at the amino terminus of the sequence SEQ ID NO: 1, 2 or 3, and/or comprising the addition of 1 amino acid, 2 amino acids, 3 amino acids, 4 amino acids, 5 amino acids, 10 amino acids, 15 amino acids, 20 amino acids, 25 amino acids, 30 amino acids, 35 amino acids, 40 amino acids, 45 amino acids, 50 amino acids, 60 amino acids, 70 amino acids, 80 amino acids, 90 amino acids, 100 amino acids, 150 amino acids, 200 amino acids, at least 500 amino acids, at least 1000 amino acids or more at the carboxy terminus of the sequence SEQ ID NO: 1, 2 or 3.

Functionally equivalent variants of a catalytic domain of sequence SEQ ID NO: 1, 2 or 3 also include sequences with a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with the sequences of SEQ ID NO: 1, 2 or 3.

The terms "identity", "identical" or "percent identity" in the context of two or more amino acid sequences, refer to two or more sequences or subsequences that are the same or have a specified percentage of amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences. One such non-limiting example of a sequence alignment algorithm is the algorithm described in Karlin et al., 1990, Proc. Natl. Acad. Sci., 87:2264-8, as modified in Karlin et al., 1993, Proc. Natl. Acad. Sci., 90:5873-7, and incorporated into the NBLAST and XBLAST programs (Altschul et al., 1991, Nucleic Acids Res., 25:3389-402). In certain embodiments, Gapped BLAST can be used as described in Altschul et al., 1997, Nucleic Acids Res. 25:3389-402. BLAST-2, WU-BLAST-2 (Altschul et al., 1996, Methods in Enzymology, 266:460-80), ALIGN, ALIGN-2 (Genentech, South San Francisco, California) or Megalign (DNASTAR) are additional publicly available software programs that can be used to align sequences. In certain embodiments, the percent identity between two nucleotide sequences is determined using the GAP program in GCG software (e.g., using a NWSgapdna.CMP matrix and a gap weight of 40, 50, 60, 70, or 90 and a length weight of 1, 2, 3, 4, 5, or 6). In certain alternative embodiments, the GAP program in the GCG software package, which incorporates the algorithm of Needleman and Wunsch (J. Mol. Biol. 48:444-53 (1970)) can be used to determine the percent identity between two amino acid sequences (e.g., using either a Blossum 62 matrix or a PAM250 matrix, and a gap weight of 16, 14, 12, 10, 8, 6, or 4 and a length weight of 1, 2, 3, 4, 5). Alternatively, in certain embodiments, the percent identity between nucleotide or amino acid sequences is determined using the algorithm of Myers and Miller (CABIOS, 4:11-7 (1989)). For example, the percent identity can be determined using the ALIGN program (version 2.0) and using a PAM120 with residue table, a gap length penalty of 12 and a gap penalty of 4. Appropriate parameters for maximal alignment by particular alignment software can be determined by one skilled in the art. In certain embodiments, the default parameters of the alignment software are used. In certain embodiments, the percentage identity "X" of a first amino acid sequence to a second sequence amino acid is calculated as 100 x (Y/Z), where Y is the number of amino acid residues scored as identical matches in the alignment of the first and second sequences (as aligned by visual inspection or a particular sequence alignment program) and Z is the total number of residues in the second sequence. If the second sequence is longer than the first sequence, then the percent identity may be determined only in the region of overlap between said first and second sequences. In this case, the same formula as above can be used but using as Z value the length of the region wherein the first and second sequence overlaps, said region having a length which is substantially the same as the length of the first sequence.

In a particular embodiment, the sequence identity is determined throughout the whole length of the sequence of the endoglucanase domain comprising the sequence of SEQ ID NO: 1, 2 or 3, or through the whole length of the variant or both.

As a non-limiting example, whether any particular polynucleotide has a certain percentage sequence identity (e.g., is at least 70% identical, at least 75% identical, at least 80% identical, at least 85% identical, at least 90% identical, and in some embodiments, at least 95%, 96%, 97%, 98%, or 99% identical) to a reference sequence can, in certain embodiments, be determined using the Bestfit program (Wisconsin Sequence Analysis Package, Version 8 for Unix, Genetics Computer Group, University Research Park, 575 Science Drive, Madison, WI 53711). Bestfit uses the local homology algorithm of Smith and Waterman, Advances in Applied Mathematics 2:482-9 (1981), to find the best segment of homology between two sequences. When using Bestfit or any other sequence alignment program to determine whether a particular sequence is, for instance, 95% identical to a reference sequence according to the present invention, the parameters are set such that the percentage of identity is calculated over the full length of the reference amino acid sequence and that gaps in homology of up to 5% of the total number of nucleotides in the reference sequence are allowed.

In some embodiments, two amino acid sequences are substantially identical, meaning they have at least 70%, at least 75%, at least 80%, at least 85%, at least 90%, and in some embodiments at least 95%, 96%, 97%, 98%, 99% amino acid residue identity, when compared and aligned for maximum correspondence, as measured using a sequence comparison algorithm or by visual inspection. Identity can exist over a region of the sequences that is at least about 10, about 20, about 40-60 residues in length or any integral value there between, and can be over a longer region than 60-80 residues, for example, at least about 90-100 residues, and in some embodiments, the sequences are substantially identical over the full length of the sequences being compared.

In a particular embodiment, suitable functionally equivalent variants of endoglucanase catalytic domain of SEQ ID NO: 1, 2 or 3 are those including one or more conservative substitutions of the amino acids. "Conservative amino acid substitutions" result from replacing one amino acid with another having similar structural and/or chemical properties For example, the following six groups each contain amino acids that are conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W). Selection of such conservative amino acid substitutions is within the skill of one of ordinary skill in the art and is described, for example by Dordo et al. et al., [J. Mol. Biol, 1999, 217;721-739] and Taylor et al., [J. Theor. Biol., 1986, 119:205-218].

In a particular embodiment, the functionally equivalent variant of the endoglucanase catalytic domain of SEQ ID NO: 1 does not comprise a deletion or substitution of the following amino acid residues of SEQ ID NO: 1: E136, E224, W174 and W258.

A functionally equivalent variant of the endoglucanase catalytic domain of sequence SEQ ID NO: 1, 2 or 3 substantially maintains or improves its endoglucanase catalytic activity.

The term "endoglucanase catalytic activity", as used herein, refers to the ability of hydrolysing 1,4-linkages in β-D-glucans also containing 1,3-linkages. Assays to determine the function of an enzyme are known by the skilled person and include, without limitation, initial rate assays, progress curve assays, transient kinetics assays and relaxation assays. Continuous assays of enzymatic activity include, without limitation, spectrophotometric, fluorometric, calorimetric, chemiluminiscent, light scattering and microscale thermopheresis assays. Discontinuous assays of enzymatic activity include, without limitation, radiometric and chromatographic assays. As the skilled person understands, factors that may influence enzymatic activity comprise salt concentration, temperature, pH, and substrate concentration. Particularly, the endoglucanase catalytic activity may be measured by means of a number of techniques assays that are conventional to the skilled person, including the viscosimetric methods using soluble derivatised cellulose, or by employing soluble or insoluble (crosslinked) dyed cellulose or mixed-linkage β-glucan, such as the carboxymethyl cellulose (CMC) assay, and the hydroxyethylcellulose (HEC) assay. In general, assays based on the use of dyed polysaccharides are standardised against a reducing sugar method that employs either CM-cellulose or β-glucan as substrate. In a particular embodiment, the CELLG3 assay (particularly K-CellG3, Megazyme International Ireland) may be used for specific endocellulases and measures activity.

As with other enzymes, the catalytic activity of the endoglucanase catalytic domain depends on a number of reaction parameters, including temperature and pH. Thus, in one embodiment, the functionally equivalent variant of the endoglucanase catalytic domain of sequence SEQ ID NO: 1, 2 or 3 maintains or improves its endoglucanase catalytic activity at a temperature of at least 0°C, at least 5°C, at least 10°C, at least 15°C, at least 20°C, at least 25°C, at least 30°C, at least 35°C, at least 37°C, at least 40°C, at least 45°C, at least 50°C, at least 55°C, at least 60°C, at least 65°C, at least 70°C, at least 75°C, at least 80°C, at least 85°C, at least 90°C, at least 95°C, at least 100°C, or higher. Likewise, in another embodiment the functionally equivalent variant of the endoglucanase catalytic domain of sequence SEQ ID NO: 1, 2 or 3 maintains or improves its endoglucanase catalytic activity at pH 0, or at least pH 0.1, or at least pH 0.5, or at least pH 1.0, or at least pH 1.5, or at least pH 2.0, or at least pH 2.5, or at least pH 3.0, or at least pH 3.5, or at least pH 4.0, or at least pH 4.5, or at least pH 5.0, or at least pH 5.5, or at least pH 6.0, or at least pH 6.5, or at least pH 7.0, or at least pH 7.5, or at least pH 8.0, or at least pH 8.5, or at least pH 9.0, or at least pH 9.5, or at least pH 10.0, or at least pH 10.5, or at least pH 11.0, or at least pH 11.5, or at least pH 12.0, or at least pH 12.5, or at least pH 13.0, or at least pH 13.5, or pH 14. All possible combinations of temperatures and pH are also contemplated by the invention.

In a particular embodiment, the functionally equivalent variant of the endoglucanase catalytic domain of SEQ ID NO: 1, 2 or 3 maintains at least 50%, at least 60%, at least 70%, at least 75%, at least 0%, at least 85%, at least 90%, at least 91%, at least 92%, at least 93%, at least 94%, at least 95%, at least 96%, at least 97%, at least 98%, at least 99%, or at least 100% of the endoglucanase catalytic activity of the catalytic domain of sequence SEQ ID NO: 1, 2 or 3; or improves it in at least 1%, at least 2%, at least 3%, at least 4%, at least 5%, at least 6%, at least 7%, at least 8%, at least 9%, at least 10%, at least 15%, at least 20%, at least 25%, at least 30%, at least 35%, at least 40%, at least 45%, at least 50%, at least 55%, at least 60%, or at least 65%, at least 70%, at least 75%, at least 80%, at least 85%, at least 90%, at least 95%, at least 100%, at least 150%, at least 200%, at least 300%, at least 400%, at least 500%, at least 1000%, or more.

In a particular embodiment, the functionally equivalent variant of the endoglucanase catalytic domain of SEQ ID NO: 1, 2 or 3 comprises or consist of an amino acid sequence with a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with the sequences of SEQ ID NO: 1, 2 or 3, and maintains at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% of the endoglucanase catalytic activity of the endoglucanase catalytic domain of sequence SEQ ID NO: 1, 2 or 3, or improves it in at least 1%, at least 2%, at least 3%, at least 4%, at least 5%, at least 6%, at least 7%, at least 8%, at least 9%, at least 10%, at least 15%, at least 20%, at least 25%, at least 30%, at least 35%, at least 40%, at least 45%, at least 50%, at least 55%, at least 60%, or at least 65%, at least 70%, at least 75%, at least 80%, at least 85%, at least 90%, at least 95%, at least 100%, at least 150%, at least 200%, at least 300%, at least 400%, at least 500%, at least 1000%, or more.

In a particular embodiment, the functionally equivalent variant of the endoglucanase catalytic domain of sequence SEQ ID NO: 1, 2 or 3 is not the catalytic domain of an endoglucanase selected from the group consisting of:

- The endocellulase shown in the UniProt database with Accession No. A0A0B2T1H3,
- The endocellulase shown in the UniProt database with Accession No. U3RD83,
- The endocellulase shown in the EMBL database with Accession No. KF240855,
- The endocellulase shown in the UniProt database with Accession No. U3R8L0,
- The endocellulase shown in the EMBL database with Accession KF240853
- The endocellulase shown in the GenSeq database with accession number BCD39860.
- The endocellulase shown in international publication WO2008005529 under SEQ ID NO:60,
- The endocellulase shown in international publication WO2008005529 under SEQ ID NO:59,
- The plant biomass degrading enzyme #67 shown in international publication WO2009208941,
- The endocellulase shown in international publication WO2011109905 under SEQ ID NO:15,
- The endocellulase shown in the UniProt database with Accession No. A0B0B2T1H3,
- The endocellulase shown in the UniProt database with Accession No. F4FAV2,
- The endocellulase shown in the UniProt database with Accession No. A0A0D0X703
- The endocellulase shown in the UniProt database with Accession No P26224.

In a particular embodiment, the enzyme comprising an endoglucanase catalytic domain further comprises a carbohydrate binding domain.

The term "carbohydrate binding domain" or "carbohydrate binding module" or "CBM", as used herein, refers to a protein domain that is present in carbohydrate-active enzymes (for example endocellulases and exocellulases) and having carbohydrate-binding activity. Carbohydrate binding domains contributes to the catalytic efficiency by increasing enzyme-substrate complex formations.

In a particular embodiment, the CBM comprises, or consists of a sequence selected from the group consisting of SEQ ID NO: 4 and 6 to 34.

In a more particular embodiment, the carbohydrate binding domain comprises or consists of SEQ ID NO: 4 or a functionally equivalent variant thereof. In an even more particular embodiment, the carbohydrate binding domain comprises or consists of the sequence of SEQ ID NO: 4.

The particulars of a functionally equivalent variant in terms of sequence identity previously described in the context of the endoglucanase catalytic domain also apply to the carbohydrate binding domain, with the necessary amendments, as will be evident for the person skilled in the art.

Thus, functionally equivalent variants of a carbohydrate binding domain of sequence SEQ ID NO: 4 also include carbohydrate binding domains comprising or consisting of amino acid sequences with a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with the sequence SEQ ID NO: 4.

In a particular embodiment, the functionally equivalent variant of the carbohydrate binding domain of SEQ ID NO: 4 maintains at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% of its carbohydrate binding activity or improves it in at least 1%, at least 2%, at least 3%, at least 4%, at least 5%, at least 6%, at least 7%, at least 8%, at least 9%, at least 10%, at least 15%, at least 20%, at least 25%, at least 30%, at least 35%, at least 40%, at least 45%, at least 50%, at least 55%, at least 60%, or at least 65%, at least 70%, at least 75%, at least 80%, at least 85%, at least 90%, at least 95%, at least 100%, at least 150%, at least 200%, at least 300%, at least 400%, at least 500%, at least 1000%, or more.

The activity of a carbohydrate binding domain may be measured as the affinity of said domain for cellulose, for example using biotinylated glycan binding assay or enzyme-linked assay (Kim et al., Biotecnhology and bioprocess engineering 19: 575-580 (2013).

In a particular embodiment, the enzyme comprises an endoglucanase catalytic domain comprising or consisting of SEQ ID NO: 1 or a functionally equivalent variant thereof that substantially maintains or improves its endoglucanase catalytic activity and a carbohydrate binding domain, preferably a carbohydrate binding domain comprising or consisting of the sequence of SEQ ID NO: 4 or a functionally equivalent variant thereof.

In a particular embodiment the enzyme comprises an endoglucanase catalytic domain comprising or consisting of SEQ ID NO: 2 or a functionally equivalent variant thereof that substantially maintains or improves its endoglucanase catalytic activity and a carbohydrate binding domain, preferably a carbohydrate binding domain comprising or consisting of the sequence of SEQ ID NO: 4 or a functionally equivalent variant thereof.

In a particular embodiment, the enzyme comprises an endoglucanase catalytic domain comprising or consisting of SEQ ID NO: 3 or a functionally equivalent variant thereof that substantially maintains or improves its endoglucanase catalytic activity and a carbohydrate binding domain, preferably a carbohydrate binding domain comprising or consisting of the sequence of SEQ ID NO: 4 or a functionally equivalent variant thereof.

In a particular embodiment, the endoglucanase catalytic domain and the carbohydrate binding domain are connected by a linking domain.

The term "linking domain", as used herein, refers to a sequence between domains. Linkers are often composed of flexible residues like glycine and serine so that the adjacent protein domains are free to move relative to one another. Longer linkers are used when it is necessary to ensure that two adjacent domains do not sterically interfere with one another.

In a particular embodiment, the linking domain comprises or consist of the sequence of SEQ ID NO: 5 or a variant thereof, said linking domain being located between the catalytic domain and the carbohydrate binding domain.

Functionally equivalent variants of a linking domain of sequence SEQ ID NO: 5 also include amino acid sequences with a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with the sequence of SEQ ID NO: 5.

In a particular embodiment, the variant of the linking domain is a functionally equivalent variant thereof. The particulars of a functionally equivalent variant previously described in the context of the endoglucanase catalytic domain also apply to the linking domain, with the necessary amendments, as will be evident for the person skilled in the art.

In a particular embodiment, the enzyme comprises an endoglucanase catalytic domain comprising or consisting of SEQ ID NO: 1 or a functionally equivalent variant thereof that substantially maintains or improves its endoglucanase catalytic activity, a carbohydrate binding domain, preferably a carbohydrate binding domain comprising or consisting of SEQ ID NO: 4 or a functionally equivalent variant thereof, and a linking domain, preferably a linking domain comprising or consisting of the sequence of SEQ ID NO: 5 or a variant thereof.

In a particular embodiment, the enzyme comprises an endoglucanase catalytic domain comprising or consisting of SEQ ID NO: 2 or a functionally equivalent variant thereof that substantially maintains or improves its endoglucanase catalytic activity, a carbohydrate binding domain, preferably a carbohydrate binding domain comprising or consisting of SEQ ID NO: 4 or a functionally equivalent variant thereof, and a linking domain, preferably a linking domain comprising or consisting of the sequence of SEQ ID NO: 5 or a variant thereof.

In a particular embodiment, the enzyme comprises an endoglucanase catalytic domain comprising or consisting of SEQ ID NO: 3 or a functionally equivalent variant thereof that substantially maintains or improves its endoglucanase catalytic activity, a carbohydrate binding domain, preferably a carbohydrate binding domain comprising or consisting of SEQ ID NO: 4 or a functionally equivalent variant thereof, and a linking domain, preferably a linking domain comprising or consisting of the sequence of SEQ ID NO: 5 or a variant thereof.

When the enzyme comprising an endoglucanase catalytic domain further comprises a carbohydrate binding domain, preferably a carbohydrate binding domain comprising or consisting of SEQ ID NO: 4, and a linking domain, preferably a linking domain comprising or consisting of the sequence of SEQ ID NO: 5 or a variant thereof, the order of these components, from the N- terminus to the C-terminus can be endoglucanase catalytic domain, linking domain and carbohydrate binding domain, or carbohydrate binding domain, linking domain and endoglucanase catalytic domain.

The method of the invention comprises contacting the substrate comprising cellulose with the enzyme comprising an endoglucanase catalytic domain comprising the sequence SEQ ID NO: 1, 2, 3 or a functionally equivalent variant thereof under suitable conditions for hydrolyzing cellulose and under a pH greater than 5.

The term "hydrolyzing cellulose", as used herein, refers to the cleave of chemical bonds of cellulose. Therefore, the conditions suitable for hydrolyzing cellulose are those conditions, which can be easily determined by the skilled person, under which at least part of the $(1\text{-}4)\text{-}\beta\text{-}D$-glucosidic links in cellulose are cleaved.

Without wanting to be bound to any theory, it is understood that endoglucanases, and as such also the enzyme comprising an endoglucanase catalytic domain comprising the sequence of SEQ ID NO: 1, 2 or 3, hydrolyse the amorphous regions of cellulose more easily than the crystalline domains. Therefore, depending on the conditions under which the substrate comprising cellulose and the enzyme are contacted, nanocelullose particles of different length, diameter, aspect/ratio and crystallinity can be obtained in different proportions.

The substrate comprising cellulose and the enzyme comprising the endoglucanase catalytic domain are contacted under a pH between 5 and 10, particularly, about 5.5, about6.0, about6.5, about6.8, about7.0, about7.2, about7.4, about7.6, about7.8, about8.0, about8.5, about9.0, about9.5, about9.8, , more particularly between 6.5 and 7.5.

In a particular embodiment, the substrate comprising cellulose and the enzyme comprising the endoglucanase catalytic domain are contacted under a temperature between 25°C and 80°C, between 30°C and 70°C, between 40°C and 60°C, between 45°C and 55°C, for example approximately 30°C, approximately 35°C, approximately 40°C, approximately 45°C, approximately 50°C, approximately 55°C, approximately 60°C, approximately 65°C, approximately 70°C, approximately 75°C, approximately 80°C, preferably approximately 50°C.

In a particular embodiment, the sample comprising cellulose is chopped into smaller pieces to accelerate the process of enzymatic hydrolysis.

In a particular embodiment, the substrate comprising cellulose and the enzyme are mixed together in water to form a suspension.

In a particular embodiment, the substrate comprising cellulose and the enzyme are contacted for a period of time of less than 72 hours, preferably less than 48 hours. In a more particular embodiment, the substrate comprising cellulose and the enzyme are contacted for a period of time comprised between 1 and 48 hours, for example, 2 hours, 4, hours, 5 hours, 6 hours, 8 hours, 10 hours, 12 hours, 14 hours, 16 hours, 18 hours, 20 hours, 22 hours, 24 hours, 26 hours, 28 hours, 30 hours, 32 hours, 34 hours, 36 hours, 38 hours, 40 hours, 42 hours, 44 hours, 46 hours, or 48 hours.

In a particular embodiment, the substrate comprising cellulose and the enzyme are contacted under agitation.

Once the above period of time is completed, that is, once the desired crystalline nanocellulose is obtained, the hydrolysis reaction can be stopped, for example, by decreasing the temperature of the reaction mixture to a temperature at which no hydrolysis is performed, for example, by incubation on ice.

Once the above period of time is completed, that is, once the desired crystalline nanocellulose is obtained, the hydrolysis reaction can be subjected to sonication.

In a particular embodiment, the resulting crystalline nanocellulose can be isolated from the rest of the components of the reaction mixture, for example, substrate that has not been hydrolysed, enzyme, or other products of the reaction, such as sugars and nanocellulose fibers (CNF). In a more particular embodiment, the crystalline nanocellulose is isolated by filtration and/or centrifugation and/or any other suitable technique known by the skilled person. In a particular embodiment, the crystalline nanocellulose is isolated by filtration. In another particular embodiment, the crystalline nanocellulose is isolated by centrifugacion. In another particular embodiment, the crystalline nanocellulose is isolated by filtration and centrifugation.

The filtration can be performed by using microfiltration, a rotatory drum filter or using a nylon filter of 5 to 20 μm mesh. To reach the maximum recovery of cellulose nanocrystals, the retentate can be resuspended in a lower volume of water and washed several times, repeating the filtration until the filtrate is clear. The cellulose nanocrystals can be recovered from the filtrate and washings by centrifugation at 15000 g (relative centrifuge force) for 30 minutes to 1 hour.

The isolation by centrifugation can be performed in two steps, a first step to wash the undigested fibers, recovering the nanocrystalline cellulose from the supernatant of successive washings with decreasing volumes of water followed by centrifugation at 4000 g (relative centrifuge force) for 5 minutes. The washings should be repeated suspending the sediment in water until the supernatant is clear and free of nanocellulose material. The second recovery step concentrates the suspended material of the supernatant of previous washings by higher speed centrifugation at 15000 g for 30 minutes to 1 hour. After centrifugation, the sediment comprising the crystalline nanocellulose can be dried, for example by spray drying or lyophilisation.

The method of the invention can optionally comprise one or more mechanical treatments to aid in the hydrolysis of the substrate by the enzyme. However, in a particular embodiment, the method does not comprise a step of microfluidization, microwaving, wet disk milling, mechanical shearing and/or bleaching.

The term "microfluidization", as used herein, refers to a process of physical disruption via two high pressure streams of liquid at high velocities.

The term "microwaving", as used herein, refers to treating a substance with electromagnetic radiation with wavelengths ranging from about one meter to one millimetre and frequencies between 300 MHz and 300 GHz. In a particular embodiment, the method of the invention does not comprise a step of microwaving the substrate comprising cellulose and the enzyme, more particular for a period of time comprised between 10 and 180 minutes, for example, between 20 and 150 minutes, between 30 and 120 minutes, between 45 and 90 minutes, between 50 and 60 minutes.

The term "wet disk milling" or "WDM", as used herein, refers to a mechanical size reduction process that fibrillates the plant cell wall to nanoscale by the application of shear force and pressure under wet conditions. In a particular embodiment, the method of the invention does not comprise a step of treating the substrate comprising cellulose by wet disk milling, in particular, by wet disk milling with a disk clearance of 10 μm at a rotational speed of 1800 rpm.

The term "mechanical shearing", as used herein, refers to a process that directly or indirectly causes breakage of particles or limits the growth of particles.

The term "bleaching", as used herein, refers to treating a substance in order to lighting its colour or whitening it. The term bleaching includes oxidative bleaching, which not only removes coloured substances, but also residual lignin and other debris, and reductive bleaching, which does not cause the loss of fiber components. Oxidative bleaching is usually carried out using sodium hypochlorite, sodium chlorite or sulfuric acid. Reductive bleaching is usually done with sodium hydrosulphite. In a particular embodiment, the method of the invention does not comprise a step of bleaching the substrate comprising cellulose or the product obtained after contacting this substrate with the enzyme comprising the endoglucanase catalytic domain. In a more particular embodiment, the method of the invention does not comprise a step of bleaching the substrate comprising cellulose or the product obtained after contacting this substrate with the enzyme comprising the endoglucanase catalytic domain with NaOH and or NaClO$_2$.

In a particular embodiment, the method of the invention does not comprise any step directed to the chemical hydrolysis of the substrate comprising cellulose, and in particular, it does not comprise treating the substrate comprising cellulose with sulphuric acid and/or with hydrochloric acid.

In a particular embodiment, the enzyme comprising an endoglucanase catalytic domain is immobilized in a solid support. Non-limiting exemplary solid supports include polymers (such as agarose, sepharose, cellulose, nitrocellulose, alginate, Teflon, latex, acrylamide, nylon, plastic, polystyrene, silicone, polymethylmetacrylate (PMMA), etc.), glass, silica, ceramics, and metals. Such solid supports may take any form, such as particles (including microparticles), sheets, dip-sticks, gels, filters, membranes, microfiber strips, tubes, wells, plates (such as microplates, including 6-well plates, 24-well plates, 96-well plates, 384-well plates, etc.), fibers, capillaries, combs, pipette tips, microarray chips, etc. In some embodiments, the surface of the solid support comprises an irregular surface, such as a porous, particulate, fibrous, webbed, or sintered surface. In some embodiments, a solid support is selected from a microplate, a microarray chip, and a microparticle. In some embodiments, a solid support is at least partially composed of a polymer.

In some embodiments, the enzyme comprising an endoglucanase catalytic domain is attached to a solid support through a linker moiety. In a particular embodiment, said linker comprises a protease cleavage site.

In a particular embodiment, the substrate comprising cellulose is a lignocellulosic material. The term "lignocellulosic material" or "lignocellulosic substrate" has been previously defined.

In a more particular embodiment, when the substrate comprising cellulose is a lignocellulosic material, the method of the invention further comprises contacting the lignocellulosic material with a xylanase and/or a lytic polysaccharide monooxygenase (LPMO).

The term "xylanase", as used herein, refers to a 1,4-beta-D-xylan-xylohydrolase (E.C. 3.2.1.8) that catalyses the endohydrolysis of 1,4-beta-D-xylosidic linkages in xylans.

Xylanase activity can be determined with 0.2% AZCL-arabinoxylan as substrate in 0.01 percent TRITON(R) X-100 and 200 mM sodium phosphate pH 6 at 37°C. One unit of xylanase activity is defined as 1.0 μmol of azurine produced per minute at 37°C, pH 6 from 0.2% AZCL-arabinoxylan as substrate in 200 mM sodium phosphate pH 6.

Xylanases (e.g. endo- beta -xylanases (E.C. 3.2.1.8), which hydrolyse the xylan backbone chain, can be from bacterial sources (e.g., Bacillus, Streptomyces, Clostridium, Acidothermus, Microtetrapsora or Thermonospora) or from fungal sources (Aspergillus, Trichoderma, Neurospora, Humicola, Penicillium or Fusarium (See, e.g., EP473 545; U.S. Pat. No. 5,612,055; WO 92/06209; and WO 97/20920)). Xylanases useful in the invention include commercial preparations (e.g., MULTIFECT(R) and FEEDTREAT(R) Y5 (Danisco Genencor), RONOZYME(R) WX (Novozymes A/S) Pulpzyme(R) HC (Novozymes A/S) and NATUGRAIN WHEAT(R) (BASF). In some embodiments the xylanase is from *Trichoderma reesei* or a variant xylanase from *Trichoderma reesei,* or the inherently thermostable xylanase described in EP1222256B1, as well as other xylanases from *Aspergillus niger, Aspergillus kawachii, Aspergillus tubigensis, Bacillus circulans, Bacillus pumilus, Bacillus subtilis, Neocallimastix patriciarum, Penicillium species, Streptomyces lividans, Streptomyces thermoviolaceus, Thermomonospora fusca, Trichoderma harzianum, Trichoderma reesei* and *Trichoderma viridae.*

In a particular embodiment, the xylanase is from *Trichoderma viridae.* The term *"Trichoderma viridae",* as used herein, refers to a fungus identified in the NCBI database by the Taxonomy ID: 5547.

The term "lytic polysaccharide monooxigenase" or "LPMO", as used herein, refers to copper-dependent enzymes that catalyse the oxidative cleave on glycosidic bonds in polysaccharides such as chitin, cellulose and hemicellulose by oxidation. LPMO oxidation is proposed to produce chain cleavage, the chain break is made on C1 or C4 carbon of glucose, even in C6 in some cases. Although originally classified as glycoside hydrolases, these enzymes are currently classified in the auxiliary activity families AA9, AS10 and AA11 in the CAZy database (Levasseur A. et al., Biotechnol Biofuels. 2013, 6: 41).

Illustrative non-limitative examples of LPMOs that can be used in the method of the invention LPMOs that oxidaze both C1 and C4, like LPMO from *Streptomyces coelicolor,* or LPMO that selectively oxidaze one of the C, like LPMO from *Myceliophthora thermophila.*

LPMO activity can be assayed using polysaccharide substrates. The assay usually comprises incubation of the substrate and the LPMO with a reductant followed by analysis of soluble products, that is, the oxidized oligosaccharides) by MALDI-TOF mass spectrometry or HPLC (high performance liquid chromatography), as described for example by Eijsink et al., Biotechnol Biofuels. 2019, 12: 58).

In a particular embodiment, the method of the invention comprises contacting the lignocellulosic material with a xylanase. In a particular embodiment, the method of the invention comprises contacting the lignocellulosic material with a lytic polysaccharide monooxygenase (LPMO). In a particular embodiment, the method of the invention comprises contacting the lignocellulosic material with a xylanase and a lytic polysaccharide monooxygenase (LPMO).

The lignocellulosic material can be contacted with the xylanase and/or LPMO previously, simultaneously or after the lignocellulosic material is contacted with the enzyme comprising an endoglucanase catalytic domain. In a preferred embodiment, the lignocellulosic material is contacted with the xylanase and/or with the LPMO at the same time it is contacted with the enzyme comprising the endoglucanase catalytic domain. In this particular embodiment, the enzyme comprising the endoglucanase domain, the xylanase and/or the LPMO can be mixed together forming an enzyme cocktail.

In a particular embodiment, the method comprises contacting the lignocellulosic material with a xylanase and/or a LPMO before or after contacting the lignocellulosic material with the enzyme comprising an endoglucanase catalytic domain under a pH greater than 5, particularly, at least 5.5, at least 6.0, at least 6.5, at least 6.8, at least 7.0, at least 7.2, at least 7.4, at least 7.6, at least 7.8, at least 8.0, at least 8.5, at least 9.0, at least 9.5, at least 10, at least 10.5, at least 11.0, at least 11.5, at least 12.0, more particularly between 6.5 and 7.5.

In a particular embodiment, the method comprises contacting the lignocellulosic material with a xylanase and/or a LPMO before or after contacting the lignocellulosic material with the enzyme comprising an endoglucanase catalytic domain under a temperature between 25°C and 80°C, between 30°C and 70°C, between 40°C and 60°C, between 45°C and 55°C, for example approximately 30°C, approximately 35°C, approximately 40°C, approximately 45°C, approximately 50°C, approximately 55°C, approximately 60°C, approximately 65°C, approximately 70°C, approximately 75°C, approximately 80°C, preferably approximately 50°C.

In a particular embodiment, the method comprises contacting the lignocellulosic material with a xylanase and/or a LPMO before or after contacting the lignocellulosic material with the enzyme comprising an endoglucanase catalytic domain under agitation.

In a particular embodiment, the method comprises contacting the lignocellulosic material with a xylanase and/or a LPMO before or after contacting the lignocellulosic material with the enzyme comprising an endoglucanase catalytic domain for a period of time of less than 72, preferably less than 48 hours, more particularly, for a period of time comprised between 1 and 48 hours, for example, 2 hours, 4, hours, 5 hours, 6 hours, 8 hours, 10 hours, 12 hours, 14 hours, 16 hours, 18 hours, 20 hours, 22 hours, 24 hours, 26 hours, 28 hours, 30 hours, 32 hours, 34 hours, 36 hours, 38 hours, 40 hours, 42 hours, 44 hours, 46 hours, or 48 hours.

In a particular embodiment, the method comprises contacting the lignocellulosic material with a xylanase and/or a LPMO before or after contacting the lignocellulosic material with the enzyme comprising an endoglucanase catalytic domain under agitation.

In a preferred embodiment, when the substrate comprising cellulose is a lignocellulosic material, the method of the invention comprises contacting simultaneously with the enzyme comprising an endoglucanase domain, with the xylanase and/or with the LPMO under a pH greater than 5, preferably between 6.5 and 7.5, under a temperature between 25°C and 80°C, preferably 50°C, and for a period of time between 1 and 48 hours, preferably about 24 hours.

*Crystalline nanocellulose*

In an aspect, the invention relates to crystalline nanocellulose, hereinafter crystalline cellulose of the invention, obtained or obtainable by the method of the invention. Therefore, all the definitions, particular and preferred embodiments of the method of the invention fully apply to the crystalline cellulose of the invention.

In a particular embodiment, the crystalline nanocellulose of the invention has a diameter comprised between 2 nm and 30 nm, for example between 5 nm and 28 nm, between 10 and 25 nm, between 15 nm and 20 nm.

In a particular embodiment, the crystalline nanocellulose of the invention has a length comprised between 10 nm and 2000 nm, for example, between 20 nm and 1800 nm, between 30 nm and 1600 nm, between 40 nm and 1400 nm, between 50 nm and 1200 nm, preferably between 50 nm and 1000 nm.

In a particular embodiment, the crystalline nanocellulose of the invention has a length to width aspect ratio comprised between 5 and 100, for example between 10 and 80, for example between 20 and 60, preferably between 20 and 100.

The diameter and length of the crystalline cellulose can be calculated using any suitable technique known by the skilled person, for example, by atomic force microscopy, as detailed in the examples herein included. Atomic force microscopy (ATM) is a technique that uses the interaction between the tip and the sample attractive-repulsion forces to create a deflection in the tip permitting to infer the imagines by mapping the deflections in each point of the sample.

As explained before, the crystalline nanocellulose of the invention preserve the structure of native cellulose, and therefore it is type I cellulose.

In a particular embodiment, the crystalline nanocellulose of the invention has a crystallinity index is $\geq 70\%$,

preferably ≥ 80%, more preferably ≥ 85%.

The crystallinity index can be determined by any suitable technique known by the skilled person, for example, X-Ray diffraction (XRD) as detailed in the examples herein included. X-Ray diffraction is a technique based on the constructive interference of monochromatic X-rays and a crystalline sample.

From the diffractograms, the crystallinity index (CI%) can be calculated using the Segal equation:

$$\text{Crystallinity index (\%)} = (I_{200}\text{-}I_{am})/I_{200} \times 100$$

where $I_{200}$ is the intensity of the cellulose crystalline peak and $I_{am}$ is the intensity of the amorphous peak.

In a particular embodiment, the crystalline nanocellulose of the invention as a crystallite size comprised between 4.0 and 7.0 nm, for example between 4.2 and 6.5 nm, between 4.5 nm and 6 nm, between 4.8 and 5.5 nm, preferably between 4.2 nm and 6.5 nm, more preferably about 5.0 nm.

The crystallite size can be determined by any suitable method known by the skilled person, for example, by X-Ray diffraction as detailed in the examples herein included. The crystallite size can be measured from the diffractograms using the Scherrer's equation:

$$\beta = \kappa\lambda/\tau cos\theta \times 100$$

where $\lambda$ is the wavelength of the incident X-ray, $\theta$ is the angle of the (200) plane, $\beta$ is the full-width at half maximum of the (200) peak, $\tau$ is the crystallite size and $\kappa$ is a constant value.

The invention will be described by way of the following examples which are to be considered as merely illustrative and not limitative of the scope of the invention.

**EXAMPLES**

**Example 1. Protein expression and purification.**

ANC EG, ANC EG+CBM, *T. maritima* EGs protein encoding genes were synthetized and codon optimized for expression in *E.coli* cells. ANC EG, ANC EG+CBM, *T. maritima* were cloned in pQE80L expression vector (Qiagen) and transformed in E.coli BL21 (DE3) (Life Technologies) for protein expression. Cells were incubated in LB medium at 37°C until OD600 reached 0.6, IPTG was added to the medium to 1 mM concentration for protein induction overnight. Cells were pelleted by centrifugation at 4000 rpm. Pellets were resuspended in extraction buffer (50 mM sodium phosphate, pH 7.0, 300 mM NaCl) and mechanically lysed using French Press. Cell debris was separated by ultracentrifugation at 33000 G for 1 hour. For purification, the supernatants were mixed with His GraviTrap affinity column (GE Healthcare) and eluted in elution buffer (50 mM sodium phosphate, pH 7.0, 300 mM NaCl, 150 mM Imidazole). Proteins were further purified by size exclusion chromatography using a Superdex 200 HR column (GE Helthcare) and eluted in 50 mM citrate buffer pH 4.8. For protein purification verification sodium dodecyl sulfate-polyacrylamide gel electrophoresis (SDS-PAGE) was used with 12% gels. The protein concentration was calculated by measuring the absorbance at 280 nm in Nanodrop 2000C.

**Example 2. Production of enzymatic nanocellulose.**

The isolation of nanocellulose using enzymes requires the incubation of an EG with a cellulosic substrate such as filter paper that contains both crystalline and amorphous regions. The ANC EG used to hydrolyze the cellulose has the sequence of SEQ ID NO: 1 and belongs to the last common ancestor of firmicutes (LFCA) and was obtained from a phylogeny using modern sequences from the family Cel5A of bacterial EG enzymes. The ANC EG displayed high activity over a wide range of conditions and in several substrates. This enzyme can be used in two forms, only the catalytic domain and linking the catalytic domain to a carbohydrate binding module (CBM). In nature, most bacterial EG have a CBM that helps in the degradation of cellulose over recalcitrant substrates. However, the CBM is poorly conserved across species and the reconstruction of ancestral from of it was not possible. In the present invention, two enzymes were obtained : (i) an enzyme including just the endoglucanase catalytic domain (ANC EG) and (ii) an enzyme including the endoglucanase catalytic domain and the CBM belonging to *Bacillus subtilis,* the species used as query sequence in the reconstruction (ANC EG+CBM). Three different modern EGs from *T. maritima, B. subtilis* and *T. reesei* where also used to compare their efficiency to that of the ancestral ones.

For nanocellulose isolation with endoglucanase treatment, Whatman N° 1 filter paper was used as substrate. Cellulose nanocrystals produced by sulfuric acid treatment purchased from Maine University were used for comparison, which were named as AcCNC. Cellulase from *Trichoderma reseei* was purchased from Sigma Aldrich (C2730). 0.4 g of filter paper were used for each hydrolysis, it was chopped in small squares of 1 cm2. The reaction was made in 40 ml of water and 5 mg were used of each EGs (ANC EG, ANC EG+CBM and *T. maritima, B.subtillis* and *T.reesei* EG) per gram of substrate. Hydrolysis was incubated at 50°C in agitation for different times (1, 5, 24, and 48 h). Reaction were stoped by incubation the hydrolysis on ice and then sonicated the mixtures with a micro tip sonicator UPH100H Ultrasonic Processor (Hielscher) for 25 min at 75%. Nanocellulose was isolated by several washings of the undigested material by centrifugation at 4000g during 5 minutes until the supernatant was clear. The cellulose nanocrystals were recovered from the supernatant of the hydrolysate and washings by higher speed centrifugation at 15000g for 1 hour. Sediment was resuspended in water and lyophilized in a Telstar Lyoquest for physicochemical characterization by freeze-drying for 24 hours.

Hydrolysis was carried out in water to avoid nanoparticles flocculation and to skip posterior dialysis steps to remove salts in buffers. Nanocellulose conversion was determined by weighing the lyophilized nanocellulose produced in each hydrolysis using the following equation:

$$\text{Nanocellulose yield (\%)} = \text{Nanocellulose mass/Initial cellulose mass} \times 100$$

Nanocellulose yields from different EGs hydrolysis are shown in **Figure 1a**. ANC EG+CBM achieved higher hydrolysis yields in comparison with ANC EG and also modern EGs. Nanocellulose conversion reached around 15% after 24 hours and increased up to 19% after 48 hours. ANC EG+CBM yield was around three times higher in comparison with ANC EG and eight, five and four times higher respect to modern EGs from *T. maritima, B. subtilis* and *T. reesei,* respectively. The different activities between enzymes were explained due to higher specific activity from ANC EG as previously reported. The low conversion from modern EGs may be due to water used as hydrolysis media. In general, saline buffers are needy for proper enzyme activity. In addition, the temperature of the hydrolysis could limit EG efficiency. Similar temperature to that used for chemical hydrolysis was chosen for better comparison to this treatment.

**Example 3. Characterization of enzymatic nanocellulose morphology by atomic force microscopy.**

Images were captured at room temperature, in tapping mode, using a Nanoscope V scanning probe microscope (Multimode 8 Bruker Digital instruments) with an integrated force generated by cantilever/silicon probes. The applied resonance frequency was 320 kHz. The cantilever had a tip radius of 5-10 nm and was 125 $\mu$m long. The samples were prepared by spin-coating (Spincoater P6700) at 2000 rpm for 130 s by casting a droplet of nanocellulose suspension on mica substrate. AFM height and phase images were collected simultaneously from all the samples. From the images the length and the diameter of 100 nanoparticles for each sample were measured to calculate the average of the length and the diameter and the aspect ratio (Length/Diameter).

Nanocellulose morphology was analyzed by Atomic Force Microscopy (AFM) from the suspensions produced during EG hydrolysis (Fig 1d and Table 1). Images were captured at room temperature, in tapping mode, using a Nanoscope V scanning probe microscope (Multimode 8 Bruker Digital instruments) with an integrated force generated by cantilever/silicon probes. The applied resonance frequency was 320 kHz. The cantilever had a tip radius of 5-10 nm and was 125 $\mu$m long. The samples were prepared by spin-coating (Spincoater P6700) at 2000 rpm for 130 s by casting a droplet of nanocellulose suspension on mica substrate. AFM height and phase images were collected simultaneously from all the samples. From the images the length and the diameter of 100 nanoparticles for each sample were measured to calculate the average of the length and the diameter and the aspect ratio (Length/Diameter).

After 1 and 5 hours of ANC EG hydrolysis, long thin fibers with lengths around 1-2 $\mu$m and diameters around 30 nm were observed. This morphology corresponded to CNF. When hydrolysis was continued for 24 hours, smaller particles with CNC dimensions, lengths around 540 nm and diameters of 20 nm were observed. ANC EG+CBM hydrolysis produced smaller particles in comparison with ANC EG. At 1 hour hydrolysis CNF and CNC mixture was observed, and after 24 hours hydrolysis a homogenous CNC suspension was obtained. These CNCs had an average length of 408 nm and diameters of 12.5 nm. After 48 hours hydrolysis bigger particles in both cases was observed due to CNC aggregations (**Fig 1b**). CNCs produced with this method have not charge in their surfaces and they tend to agglomerate. Enzyme activity started to saturate after 24 hours (**Fig 1a**). EG from *T. maritima* produced bigger particles in length and diameter due to low substrate digestion. EG from *T. reesei* produced similar suspension to ANC EG and *B. subtilis* EG produced particles with size smaller than *T. maritima* EG but bigger than ancestral endoglucanases. The size population distribution from all enzymatic hydrolysis was plotted to analyze the suspension homogeneity by measuring 100 particles of each suspension (**Fig 2**). ANC EG+CBM achieved the more homogenous populations as AFM images showed. Any modern enzyme reached the same homogeneous population reached by ANC EG+CBM after 24 hours hydrolysis (**Fig 2**).

**Table 1.** Average length and diameter and L/D aspect ratio of the nanocellulose produced at different hydrolysis times with ANC EG, ANC EG+CBM, *T. maritima, B.subtillis* and *T. reesei* EGs using filter paper as a substrate, and the nanocellulose produced by acid sulfuric treatment. The average and S.E.M (standard error mean) were calculated with the lengths and diameters of 100 particles each condition.

| Enzyme | Time (hours) | Lenght (nm) | Diameter (nm) | L/D |
|---|---|---|---|---|
| ANC EG | 1 | 1476.8±1003.2 | 31.0±16.6 | 47.6 |
| | 5 | 960.0±821.8 | 25.3±15.0 | 37.9 |
| | 24 | 540.8±460.4 | 20.8±10.0 | 26.0 |
| | 48 | 856.3±804.5 | 21.7±11.4 | 39.5 |
| | 72 | 748.5±561.1 | 22.7±11.2 | 34.5 |
| ANC EG+CBM | 1 | 1017.6±670.8 | 23.0±11.6 | 44.2 |
| | 5 | 482.3±207.2 | 19.0±9.0 | 25.4 |
| | 24 | 408.0±166.9 | 12.5±7.0 | 32. 6 |
| | 48 | 933.3±1130.2 | 17.5±19.7 | 53.3 |
| | 72 | 726.0±614.3 | 19.0±9.7 | 38.2 |
| *T. maritima* EG | 1 | 1872.3±1245.2 | 40.0±40.0 | 46.8 |
| | 5 | 1919.2±1083.4 | 31.3±20.0 | 61.3 |
| | 24 | 1275.7±945.0 | 23.0±16.0 | 55.5 |
| | 48 | 970.0±686.7 | 27.9±20.8 | 34.8 |
| | 72 | 1416.0±989.3 | 25.5±17.5 | 55.5 |
| AcCNC | - | 173.9±57.2 | 10.0±3.2 | 17.3 |

The EnCNC suspension was compared with a commercial sample produced by sulfuric acid hydrolysis (AcCNC). As expected, the suspension from the ANC EG+CBM was the more similar to AcCNC (**Fig 1c**).

The morphology of EnCNC and AcCNC was further analyzed. AFM images show the different shapes from both samples (**Fig 1e**). EnCNC produced after 24 hours with ANC EG+CBM was longer but maintained similar diameters to AcCNC. This difference in size was due to aggressiveness of acid hydrolysis in comparison to enzyme activity. EnCNC showed a needle-like morphology due to its high aspect ratio, 32. AcCNC had a ribbon-like and its aspect ratio was 17. Bases on these morphologies, that were reported in the literature from different cellulose polymorphs, EnCNC can be assigned to cellulose I and AcCNC to cellulose II (De Souza Lima, M. M. and Borsali, R., Macromol. Rapid Commun. 2004, 25, 771-787). These results suggested that enzymatic treatment does not modify the native cellulose structure of filter paper, whereas acid hydrolysis could affect cellulose chain arrangement, transforming cellulose I to cellulose II.

**Example 4. Characterization of enzymatic nanocellulose structure by Fourier transform infrared spectroscopy and Nanoscale-resolved Fourier transform infrared spectroscopy**

The physicochemical properties of CNC can be determined by the isolation method. To fully characterize these properties an unprecedented arsenal of techniques including Fourier Transform Infrared Spectroscopy (FTIR); nano-FTIR, which combines FTIR and scanning near-filed optical microscopy (s-NON); X-ray Powder Diffraction (XRD); Solid-state Cross Polarization Magnetic Angle Spinning Carbon-13 Nuclear Magnetic Resonance (CP/MAS $^{13}$C NMR) and Thermogravimetric Analysis (TGA) was set up. Using these techniques EnCNC was compared to both the cellulosic substrate filter paper and commercial AcCNC.

Fourier transform infrared spectroscopy (FTIR) was used for studying cellulose functional groups spectra. FTIR spectroscopy measurements were recorded by a Nicolet Nexus spectrometer provided with a MKII Golden Gate accessory (Specac) with a diamond crystal at a nominal incidence angle of 45° and ZnSe lens. Spectra were measured in attenuated reflection (ATR) mode between 4000 and 650 cm-1, with averaging 32 scans and a resolution of 4 cm-1. Scattering-type scanning near-field optical microscopy (IR s-SNOM) and nanoscale-resolved Fourier transform infrared (nano-FTIR) permit to study the infrared spectra from a single nanocellulose particle of a suspension. The IR s-SNOM images and nano-FTIR spectra, as well as the corresponding AFM images, were recorded with a near SNOM system (Neaspec GmbH, Germany) comprising both s-SNOM and nano-FTIR capabilities. Pt-Si coated AFM tips were used. Nano-FTIR spectroscopy of the nanocellulose suspension was performed with illumination from a mid-infrared laser supercontinuum, using Au coated AFM tips. The final nano-FTIR spectrum was obtained by averaging 5 individual spectra. The total acquisition time was 5 min and the spectral resolution 16 cm-1. The spectra were normalized to that obtained on a clean gold surface (reference measurements).

FTIR spectra measurements allow to study cellulose structure and its functional groups (**Fig. 3b**). Filter paper spectrum shows the typical functional groups from native cellulose. The wide band between 3600 and 3100 $cm^{-1}$ corresponded to -OH groups stretching vibration. At 2900 $cm^{-1}$ appeared the band of -CH and $-CH^2$ groups stretching vibration. The spectrum presents a peak at 1640 $cm^{-1}$ attributed to the bending mode of absorbed water. Also, the spectrum had the characteristic peaks of cellulose at 1425, 1364, 1335, 1316, 1227, 1203 and 1158 $cm^{-1}$. The peak at 897 cm-1 is attributed to the C-O asymmetric stretching of β-linkages between glucose monomers, related with cellulose rotation around β-glycosidic linkages. This spectrum is typical of cellulose I polymorph. Enzymatic hydrolysis does not generate any dramatic change in cellulose structure at different reaction times (**Fig 4 and 5**). The main peaks were maintained in all EnCNC spectra and no band in the Amide II interval around 1540 $cm^{-1}$ is observed, suggesting no protein residues and highly pure nanocellulose samples. Although samples were freeze-dried prior characterization, the water absorption peak is maintained due to strong cellulose-water hydrogen bonding interaction. Both, filter paper and EnCNC spectra show the typical peak of cellulose Iβ at 710 cm-1, suggesting that these samples have cellulose Iβ structure. Indeed, the peak at 750 $cm^{-1}$ from Cellulose Iα polymorph is not observed.

AcCNC spectrum **(Fig 1b and 6)** presents some differences, two small shoulders can be observed at 3486 $cm^{-1}$ and 3441 $cm^{-1}$ assigned to the intra-molecular hydrogen bonding in the allomorph cellulose I. The peak at 2894 $cm^{-1}$ was sharper in AcCNC sample in comparison with EnCNC, this shape is typical from crystal structure of cellulose II. The band at 1425 $cm^{-1}$ that corresponds to $-CH_2$ bending was smaller in AcCNC indicating new inter and intra molecular hydrogen bonds. In addition, a shift in the β-glycosidic peak was observed, in EnCNCs and filter paper appeared at 897 $cm^{-1}$, while in the spectra of AcCNC it appeared at 895 $cm^{-1}$, and was sharper than that of EnCNC. This alteration is typical from cellulose II. A second derivative analysis from FTIR spectra was performed for further analysis (**Fig 7**), in the range from 3000 to 2850 $cm^{-1}$, some shifts in EnCNCs spectra in comparison to AcCNC sample where observed, due to new inter and intra molecular interactions and small torsion in the angle of the β-glycosidic bonds of AcCNC. Moreover, in the range from 1000 to 750 $cm^{-1}$, the shift to 895 $cm^{-1}$ of the β-glycosidic peak in AcCNC sample was observed. Inversely, the spectra presented similar pattern in the range from 1500 to 1100 $cm^{-1}$. These differences corroborate the changes in cellulose chain arrangement between polymorphs. A typical chemical signature of AcCNC is sulfate groups from the acidic hydrolysis that could be observed in the spectrum as a peak at 814 cm-1. Sulfuric acid hydrolysis disrupts the cellulose structure degrading the original parallel chain crystalline structure from cellulose I to an antiparallel orientation in cellulose II 24. This sulfate groups are anchored in the nanocrystals surface during hydrolysis by an esterification reaction.

The infrared spectra was also investigated using nano-FTIR that allows infrared analysis with nanoscale resolution, but it has not been used much in CNC characterization. An EnCNC suspension was analyzed to obtain spectra from different nanocrystals. Figure 3c shows an AFM image from EnCNC suspension prepared after 5 hours of hydrolysis with ANC EG+CBM. The aim was to find an area that contained crystals of different sizes to see if size affects nano-FTIR resolution. The circles in **Figure 3c** mark the crystals selected for the measurement and the spectrum for each crystal is presented in **Figure 3d.** Larger crystals spectra have better resolution than the smaller ones. In all cases two prominent peaks were observed at 1158 $cm^{-1}$, associated to the C-O-C stretching of the β-1,4-glycosidic linkage, and the 1052 $cm^{-1}$ band that corresponds to C-O bending peak. For comparison, the average spectrum obtained by nano-FTIR was plotted with the one measured using Attenuated Total Reflectance FTIR (ATR-FTIR) (**Figure 7**). In the nano-FTIR spectrum a simplified version of the spectrum is seen, losing some peaks like the peak at 897 $cm^{-1}$ of β-glycosidic bond, and the peaks at 1223 and 1028 $cm^{-1}$. In general, the most important peaks were observed in both spectra. The spectrum taken by nano-FTIR was similar to the one measured from CNCs produced by sulfuric acid hydrolysis in a previous work .

**Example 5. Determination of the sulfur content of enzymatic and acid crystalline nanocelulloses by conductometric titration**

Sulfate groups have a direct impact in the physicochemical properties of nanocrystals. For example, water dispersability from both EnCNC and AcCNC suspensions was different due to the functional groups in the crystal surface (**Figure 3a**). -OH groups on EnCNC surface induced interaction between crystals by hydrogen bonding, as observed in the AFM analysis, which translate into an opaque solution. The negative charged $OSO_3^-$ groups in AcCNC surface leads to a stable dispersion in water due to electrostatic repulsion forces between crystals.

Sulfate content on AcCNC was measured by conductometric titration at 25°C with a Crison ECMeter GLP 31 conductometer that was calibrated with 147, 1413 and 12.88 $\mu$S/cm standards. AcCNC powder was diluted in 25 ml of water and sonicated for 30 min, then 10 ml of 10 mM HCl were added. The dispersion was continuously titrated with 10 mM NaOH 25. NaOH consumption was measured for the sample titration and the blank, and the sulfur content was calculated using the equation:

$$(\%) = (32 \times M \times V) \times 100$$

where M is the NaOH concentration, V is the volume of NaOH solution that needed to titrate the sample (without the volume used to titrate the blank) and $\omega$ is the weight of AcCNC used.

The percentage of sulfur in AcCNC was quantified by conductometric titration (**Fig. 8**), measuring $0.95\pm0.04$ sulfur content, in agreement with the 0.94% reported by the manufacturer.

**Example 6. Characterization of enzymatic nanocellulose crystalline structure by X-ray diffraction**

One of the most important features of CNC is its crystallinity because it is correlated to its thermal and mechanical properties. XRD was used to analyze CNC crystal structure and determine the Crystallinity Index (CI) (Fig. 3e), and compare the crystallinity between different CNC types. X-ray powder diffraction patterns were collected by using a Philips X'pert PRO automatic diffractometer operating at 40 kV and 40 mA, in theta-theta configuration, a secondary monochromator with Cu-Ka radiation ($\lambda$ = 1.5418 A) and a PIXcel solid state detector (active length in $2\theta$ 3.347o). Data were collected from 5 to 50o $2\theta$ (step size 0.026 and time per step 80 s) at room temperature. A fixed divergence and antiscattering slit giving a constant volume of sample illumination were used.

From the diffractograms, the crystallinity index (CI%) was calculated using the Segal equation:

$$\text{Crystallinity index } (\%) = (I_{200}-I_{am})/I_{200} \times 100$$

where $I_{200}$ is the intensity of the cellulose crystalline peak and $I_{am}$ is the intensity of the amorphous peak.

The crystallite size was measured from the diffractograms using the Scherrer's equation:

$$\beta = \kappa\lambda/\tau cos\theta \times 100$$

where $\lambda$ is the wavelength of the incident X-ray, $\theta$ is the angle of the (200) plane, $\beta$ is the full-width at half maximum of the (200) peak, $\tau$ is the crystallite size and $\kappa$ is a constant value.

From the diffractograms in **Figure 3e,** CI and crystallite size were calculated. It was observed that filter paper diffractogram presented the classical crystallography pattern from cellulose I. At 22.9° appeared a sharp peak that corresponded to (200) plane and three smaller ones at 15°, 16.5° and 34° attributed to (110), (101), (004) planes, respectively. After 24 hours of enzymatic hydrolysis with ANC EG, no change in the diffractogram pattern is observed as the (110), (101),(004) and (200) were in the same position. Enzymatic treatment keeps the native cellulose structure, in agreement with previous FTIR analysis. It was found that AcCNC pattern was different, the plane (200) is shifted to 22.3°, new peaks appear at 19.9° and 12.3° attributed to (102) and (010) planes, respectively. These two peaks are produced by transformation of crystalline structure of cellulose I to cellulose II. The two peaks at 15° and 16.5° are maintained from (110) and (101) plane but less intense, and (004) plane at 34° is sharper. This diffractogram pattern has been attributed to a mixture of cellulose I and II. It was determined that filter paper has a CI% of 92.5% and a crystallite size of 6.5 nm (Table S2). EnCNCs samples presented a CI% of 85.0% with ANC EG and 87.9% with ANC EG+CBM, and around 5 nm of crystallite size. The reduction in both parameters in comparison with filter paper may be caused by the freeze-dried process as well as small disruption in cellulose structure during EG treatment. AcCNC sample has a CI% of 80.5% and a crystallite size of 4.1 nm. Sulfuric acid treatment produced a partial transformation from cellulose I to cellulose II, forming a less crystalline polymer by breaking cellulose crystalline structure.

**Table 2.** Crystallinity index (CI%) and crystallite size of the filter paper, EnCNCs produced by 24 hours hydrolysis by ANC EG and ANC EG-CBM and AcCNCs produced by sulfuric acid hydrolysis. The CI% was calculated by Segal method and the Scherrer's equation was used for the crystallite size.

| Enzyme | CI (%) | Crystallite size (nm) |
|---|---|---|
| Filter paper | 92.5 | 6.5 |
| EnCNC ANC EG | 85.0 | 4.9 |
| EnCNC ANC EG+CBM | 87.9 | 5.1 |
| AcCNC | 80.5 | 41 |

**Example 7. Characterization of enzymatic nanocellulose chemical structure by solid-state cross-polarization magic angle spinning $^{13}$C nuclear magnetic resonance.**

To further investigate the changes in CNC observed in FTIR and XRD analysis Solid-state cross-polarization magic angle spinning $^{13}$C nuclear magnetic resonance (CP/MAS $^{13}$C NMR) was used, which permits to study the lignocellulose biomass, the cellulose structure and the effect that different degradation treatments have. $^{13}$C CP/MAS NMR spectra were recorded on a 400 MHz BRUKER system equipped with a 4 mm MASDVT TRIPLE Resonance HYX MAS probe. Larmor frequencies were 400.17 MHz and 100.63 MHz for $^{1}$H and $^{13}$C nuclei, respectively. Chemical shifts were reported relative to the signals of $^{13}$C nuclei in glycine. Sample rotation frequency was 12 kHz and relaxation delay was 5 s. The number of scans was 2k. Polarization transfer was achieved with RAMP cross-polarization (ramp on the proton channel) with a contact time of 5 ms. High-power SPINAL 64 heteronuclear proton decoupling was applied during acquisition.

All the CNC samples analyzed presented the same cellulose pattern (**Fig 3f**). The peak between 60 and 70 ppm corresponded to C6 of the primary alcohol group. Peaks between 70 and 80 ppm are assigned to the carbons C2, C3, and C5. The peak between 80 and 95 ppm is attributed to C4 and the peak from 100 to 110 ppm is the C1 anomeric carbon. These features appears in both crystalline and amorphous components of cellulose 35. In both EnCNC samples, C6 peak was sharp and appeared at 65 ppm, typical in cellulose I. C6 peak in AcCNC sample was shifted to 63 ppm, a feature from cellulose II. There is a small shift between the C4 positions, the peak appears at 89 ppm in EnCNC and filter paper, and corresponds to cellulose I. In AcCNC, this peak is found at 88 ppm, which is the position in cellulose II. Also, in EnCNCs spectra the C4 band is sharper; this means that the anhydroglucose units is in a very ordered environment attributed to the crystalline cellulose region. The shoulder at 84 ppm is assigned to a more heterogeneous distribution in the structural order; this is typical of the anhydroglucoses located at the surface. The reduction in the areas of C1 and C6 in AcCNC can be associated to the lower crystallinity. These results match with the XRD data and confirm the partial transformation to cellulose II in AcCNC due to other typical features of cellulose II weren't found in the spectra, for example the splitting in C1 and C4 peak from cellulose II.

**Example 8. Determination of the thermal stability of enzymatic nanocellulose by thermogravimetric analysis.**

After determining the chemical and structural features of EnCNC the thermal stability was investigated, which has interest from the application of CNC. The thermostability can be determined using thermogravimetric analysis (TGA), which provides a measure of the weight loss (**Fig. 3g**). The analysis was performed using TGA/SDTA 851 Mettler Toledo equipment. Up to 10 mg of the samples were used and were heated from 30 to 800°C in a nitrogen atmosphere at a scanning rate of 10°C/min. The initial degradation temperature (To) is described as the loss of 5% of the weight of the total sample and the maximum degradation temperature (Td) is the minimum of the degradation peak in the derivative of thermogravimetric curves (DTG). The derivatives of the thermogravimetric curves (DTG) for filter paper, EnCNCs and AcCNC are shown in **Figure 3h.** In **Table 3,** the onset degradation temperature (To), the maximum degradation temperature (Td) and char weight (%) are shown. All the samples present a weak weight loss around 100°C that corresponded to water evaporation that was absorbed by the material. Filter paper has the classical degradation behavior of native cellulose, starting at 311°C (To) with maximum degradation temperature at 361°C, as observed in the DTG curve. EnCNC samples show a different thermal behavior. EnCNC produced by ANC EG has a To of 210°C and a Td of 337°C. In the case of EnCNC produced by ANC EG+CBM a higher To and Td, 262 and 356°C, respectively, were found. This variation could be explained by the different catalytic action between EGs. ANC EG acts randomly in the fiber in comparison with ANC EG+CBM that is processive. This translates into a more heterogeneous length population with ANC EG in which larger particles have more amorphous regions. In fact, it has been suggested that cellulose degradation starts in the amorphous regions. Also, smaller nanocrystals have lower polymerization degree that leads to lower thermal stability. Nevertheless, it is worth noting that EnCNC obtained by hydrolysis with ANC EG+CBM has nearly the Td of the substrate filter paper. This contrast with the thermal stability of AcCNC that has a To of 269°C and a Td of 298°C. Although it has been described that cellulose II is more thermostable than cellulose I, the amount of sulfate groups on the crystal surface of AcCNC clearly decreases the thermal stability. The lower crystallinity and polymerization degree of AcCNC also likely play a role in the lower To and Td.

**Table 3.** Onset degradation temperature (To), maximum thermal degradation temperature (Td) and char residue (%) of the filter paper, EnCNCs produced by 24 hours hydrolysis with ANC EG and ANC EG+CBM, and AcCNCs produced by sulfuric acid hydrolysis.

| Enzyme | To (°C) | Td (°C) | Char residue (wt%) |
|---|---|---|---|
| Filter Paper | 311 | 361 | 9.2 |
| EnCNC ANC EG | 210 | 337 | 14.2 |

(continued)

| Enzyme | To (°C) | Td (°C) | Char residue (wt%) |
|---|---|---|---|
| **EnCNC ANC EG+CBM** | 262 | 356 | 13.9 |
| **AcCNC** | 269 | 298 | 22.2 |

Overall, these results show that the enzymatic method maintain native cellulose structure as well as its properties. EnCNC show slightly lower crystallinity and thermal stability than those of filter paper used as substrate. Sulfuric acid hydrolysis had a stronger effect in cellulose structure, partially transforming cellulose chain arrangement, degrading the cellulose crystalline structure, and even changing the functional groups on the crystal surface by anchoring sulfate groups that seems to reduce AcCNC thermal stability.

**Claims**

1. A method for producing crystalline nanocellulose comprising contacting a substrate comprising cellulose with an enzyme comprising an endoglucanase catalytic domain under suitable conditions for hydrolyzing cellulose and under a pH greater than 5, wherein the endoglucanase catalytic domain comprises a sequence selected from the group consisting of SEQ ID NO: 1, SEQ ID NO: 2, SEQ ID NO: 3 and a functionally equivalent variant thereof that substantially maintains or improves its endoglucanase catalytic activity.

2. The method according to claim 1, wherein the enzyme comprising an endoglucanase catalytic domain further comprises a carbohydrate binding domain.

3. The method of claim 2, wherein the carbohydrate binding domain comprises the sequence of SEQ ID NO: 4 or a functionally equivalent variant thereof.

4. The method according to any one of claims 1 to 3, wherein the endoglucanase catalytic domain and the carbohydrate binding domain are connected by a linking domain.

5. The method according to claim 4, wherein the linking domain comprises the sequence of SEQ ID NO: 5 or a variant thereof, said linking domain being located between the catalytic domain and the carbohydrate binding domain.

6. The method according to any one of claims 1 to 5, wherein the suitable conditions for hydrolysing cellulose comprise contacting the substrate and the enzyme at a temperature of approximately 50°C.

7. The method according to any one of claims 1 to 6, wherein the suitable conditions for hydrolysing cellulose comprise contacting the substrate and the enzyme for a period of time of less than 48 hours.

8. The method according to claim 7, wherein the period of time is between 5 and 48 hours.

9. The method according to any one of claims 1 to 8, wherein the enzyme comprising an endoglucanase catalytic domain is immobilized in a solid support.

10. The method according to any one of claims 1 to 9, further comprising isolating the crystalline nanocellulose.

11. The method according to claim 10, wherein the crystalline nanocellulose is isolated by filtration and/or centrifugation.

12. The method according to any one of claims 1 to 11, wherein the substrate comprising cellulose is paper pulp.

13. The method according to any one of claims 1 to 12, wherein the substrate comprising cellulose is a lignocellulosic material.

14. The method according to claim 13, further comprising contacting the lignocellulosic material with a xylanase and/or with a lytic polysaccharide monooxygenase (LPMO).

15. Crystalline nanocellulose obtainable by the method according to any one of claims 1 to 14.

A

B

FIG. 1

C

D

E

EnCNC          AcCNC

FIG. 1 (CONT.)

**FIG. 2**

FIG. 2 (CONT.)

FIG. 3

**FIG. 3 (CONT.)**

**FIG. 4**

FIG. 5

**FIG. 6**

FIG. 7

FIG. 8

Europäisches
Patentamt

European
Patent Office

Office européen
des brevets

## EUROPEAN SEARCH REPORT

Application Number

EP 20 38 2141

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 192 866 A1 (CIC NANOGUNE - ASOCIACIÓN CENTRO DE INVESTIGACIÓN COOP EN NANOCIENCIAS) 19 July 2017 (2017-07-19) * sequences 2,3 * | 1-15 | INV. C12N9/42 C08L1/02 |
| X | EP 3 502 126 A1 (CIC NANOGUNE ASOCIACION CENTRO DE INVESTIG COOPERATIVA EN NANOCIENCIAS) 26 June 2019 (2019-06-26) * sequences 11,12 * | 1-15 | |
| A | GUNNAR HENRIKSSON ET AL: "Monocomponent endoglucanase treatment increases the reactivity of softwood sulphite dissolving pulp", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 32, no. 5, 1 May 2005 (2005-05-01), pages 211-214, XP019357727, ISSN: 1476-5535, DOI: 10.1007/S10295-005-0220-7 * figure 1 * | 1-15 | |
| A | QING YAN ET AL: "A comparative study of cellulose nanofibrils disintegrated via multiple processing approaches", CARBOHYDRATE POLYMERS, APPLIED SCIENCE PUBLISHERS, LTD. BARKING, GB, vol. 97, no. 1, 4 May 2013 (2013-05-04), pages 226-234, XP028568606, ISSN: 0144-8617, DOI: 10.1016/J.CARBPOL.2013.04.086 * abstract * | 1-15 | TECHNICAL FIELDS SEARCHED (IPC) C12N C09J C08L |

-/--

The present search report has been drawn up for all claims

| Place of search | Date of completion of the search | Examiner |
|---|---|---|
| Munich | 27 May 2020 | Griesinger, Irina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 20 38 2141

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MARIA M. PÉREZ-MADRIGAL ET AL: "Powering the future: application of cellulose-based materials for supercapacitors", GREEN CHEMISTRY, vol. 18, no. 22, 1 January 2016 (2016-01-01), pages 5930-5956, XP55697295, GB ISSN: 1463-9262, DOI: 10.1039/C6GC02086K * abstract * | 1-15 | |
| A | US 2016/340520 A1 (KONAGAYA SHIGEJI [JP] ET AL) 24 November 2016 (2016-11-24) * abstract * | 1-15 | |
| A | WO 2013/076372 A1 (TEKNOLOGIAN TUTKIMUSKESKUS VTT OY [FI]) 30 May 2013 (2013-05-30) * abstract * | 1-15 | |
| A | WO 2015/200232 A2 (API IP HOLDINGS LLC [US]) 30 December 2015 (2015-12-30) * abstract * | 1-15 | **TECHNICAL FIELDS SEARCHED (IPC)** |
| A | ISTVAN SIRO ET AL: "Microfibrillated cellulose and new nanocomposite materials: a review", CELLULOSE, vol. 17, no. 3, 21 February 2010 (2010-02-21), pages 459-494, XP055068118, ISSN: 0969-0239, DOI: 10.1007/s10570-010-9405-y * abstract * | 1-15 | |

The present search report has been drawn up for all claims

| Place of search | Date of completion of the search | Examiner |
|---|---|---|
| Munich | 27 May 2020 | Griesinger, Irina |

EPO FORM 1503 03.82 (P04C01)

page 2 of 2

## ANNEX TO THE EUROPEAN SEARCH REPORT
## ON EUROPEAN PATENT APPLICATION NO.

EP 20 38 2141

This annex lists the patent family members relating to the patent documents cited in the above-mentioned European search report.
The members are as contained in the European Patent Office EDP file on
The European Patent Office is in no way liable for these particulars which are merely given for the purpose of information.

27-05-2020

| Patent document cited in search report | | Publication date | Patent family member(s) | | Publication date |
|---|---|---|---|---|---|
| EP 3192866 | A1 | 19-07-2017 | CA | 3011534 A1 | 20-07-2017 |
| | | | EP | 3192866 A1 | 19-07-2017 |
| | | | EP | 3402882 A1 | 21-11-2018 |
| | | | US | 2019062717 A1 | 28-02-2019 |
| | | | WO | 2017121902 A1 | 20-07-2017 |
| EP 3502126 | A1 | 26-06-2019 | EP | 3502126 A1 | 26-06-2019 |
| | | | WO | 2019121719 A1 | 27-06-2019 |
| US 2016340520 | A1 | 24-11-2016 | JP | WO2016043145 A1 | 03-08-2017 |
| | | | JP | WO2016043146 A1 | 03-08-2017 |
| | | | US | 2016319176 A1 | 03-11-2016 |
| | | | US | 2016340520 A1 | 24-11-2016 |
| | | | WO | 2016043145 A1 | 24-03-2016 |
| | | | WO | 2016043146 A1 | 24-03-2016 |
| WO 2013076372 | A1 | 30-05-2013 | FI | 20116175 A | 25-05-2013 |
| | | | WO | 2013076372 A1 | 30-05-2013 |
| WO 2015200232 | A2 | 30-12-2015 | US | 2015368368 A1 | 24-12-2015 |
| | | | US | 2017190800 A1 | 06-07-2017 |
| | | | US | 2019292277 A1 | 26-09-2019 |
| | | | WO | 2015200232 A2 | 30-12-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008005529 A
- WO 2009208941 A
- WO 2011109905 A
- EP 473545 A
- US 5612055 A
- WO 9206209 A
- WO 9720920 A
- EP 1222256 B1

### Non-patent literature cited in the description

- **IWAMOTO, S. et al.** *Appl. Phys. A,* vol. 220 (89), 461-466
- **HENRIKSSON, M. et al.** *Eur. Polym. J.,* 2007, vol. 43, 3434-3441
- **ZHU, J.Y. et al.** *Green Chem.,* 2011, vol. 13, 1339-1344
- **SATYARMURTHY, P. et al.** *Carbohydr. Polym.,* 2011, vol. 83, 122-129
- **YARBROUGH, J. M. et al.** *Acs Nano,* 2017, vol. 11, 3101-3109
- **DA SILVA, T.A. et al.** *BioResources,* 2007, vol. 2 (4), 616-629
- **HU, J. et al.** *Sci. Rep.,* 2018, vol. 8 (1), 3195
- **KARLIN et al.** *Proc. Natl. Acad. Sci.,* 1990, vol. 87, 2264-8
- **KARLIN et al.** *Proc. Natl. Acad. Sci.,* 1993, vol. 90, 5873-7
- **ALTSCHUL et al.** *Nucleic Acids Res.,* 1991, vol. 25, 3389-402
- **ALTSCHUL et al.** *Nucleic Acids Res.,* 1997, vol. 25, 3389-402
- **ALTSCHUL et al.** *Methods in Enzymology,* 1996, vol. 266, 460-80
- **NEEDLEMAN ; WUNSCH.** *J. Mol. Biol.,* 1970, vol. 48, 444-53
- **MYERS ; MILLER.** *CABIOS,* 1989, vol. 4, 11-7
- **SMITH ; WATERMAN.** *Advances in Applied Mathematics,* 1981, vol. 2, 482-9
- **DORDO et al.** *J. Mol. Biol,* 1999, vol. 217, 721-739
- **TAYLOR et al.** *J. Theor. Biol.,* 1986, vol. 119, 205-218
- **KIM et al.** *Biotecnhology and bioprocess engineering,* 2013, vol. 19, 575-580
- **LEVASSEUR A. et al.** *Biotechnol Biofuels.,* 2013, vol. 6, 41
- **EIJSINK et al.** *Biotechnol Biofuels,* 2019, vol. 12, 58
- **DE SOUZA LIMA ; M. M. ; BORSALI, R.** *Macromol. Rapid Commun.,* 2004, vol. 25, 771-787

SEQUENCE LISTING

```
<110>   CIC NANOGUNE – ASOCIACIÓN CENTRO DE INVESTIGACIÓN
        COOPERATIVA EN NANOCIENCIAS
        UNIVERSIDAD DEL PAÍS VASCO / EUSKAL HERRIKO UNIBERTSITATEA

<120>   METHOD FOR PRODUCING CRYSTALLINE NANOCELLULOSE

<130>   P18154EP00

<160>   34

<170>   PatentIn version 3.5

<210>   1
<211>   297
<212>   PRT
<213>   Artificial Sequence

<220>
<223>   Ancestral endoglucanase catalytic domain

<400>   1

Thr Pro Val Glu Thr His Gly Gln Leu Ser Val Lys Gly Gly Gln Leu
1               5                   10                  15


Val Asp Glu Asn Gly Lys Pro Val Gln Leu Arg Gly Met Ser Ser His
            20                  25                  30


Gly Leu Gln Trp Phe Gly Asp Phe Val Asn Lys Asp Ser Met Lys Trp
            35                  40                  45


Leu Arg Asp Asp Trp Gly Ile Asn Val Phe Arg Val Ala Met Tyr Thr
        50                  55                  60


Ala Glu Gly Gly Tyr Ile Thr Asn Pro Ser Val Lys Asn Lys Val Lys
65                  70                  75                  80


Glu Ala Val Glu Ala Ala Ile Asp Leu Gly Met Tyr Val Ile Ile Asp
            85                  90                  95


Trp His Ile Leu Ser Asp Asn Asp Pro Asn Thr Tyr Lys Glu Gln Ala
            100                 105                 110


Lys Ala Phe Phe Gln Glu Met Ala Ala Lys Tyr Gly Asn Tyr Pro Asn
            115                 120                 125


Val Ile Tyr Glu Ile Cys Asn Glu Pro Asn Gly Gly Val Thr Trp Ser
        130                 135                 140


Asn Gln Ile Lys Pro Tyr Ala Glu Glu Val Ile Pro Ala Ile Arg Ala
145                 150                 155                 160
```

```
Asn Asp Pro Asp Asn Ile Ile Ile Val Gly Thr Pro Thr Trp Ser Gln
            165             170             175

Asp Val His Asp Ala Ala Asp Asn Pro Leu Pro Tyr Ser Asn Ile Met
            180             185             190

Tyr Ala Leu His Phe Tyr Ala Gly Thr His Gly Gln Ser Leu Arg Asp
            195             200             205

Lys Ile Asp Tyr Ala Leu Ser Lys Gly Val Ala Ile Phe Val Thr Glu
        210             215             220

Trp Gly Thr Ser Asp Ala Ser Gly Asn Gly Gly Pro Phe Leu Asn Glu
225             230             235             240

Ser Gln Lys Trp Ile Asp Phe Met Asn Ser Arg Asn Ile Ser Trp Ala
            245             250             255

Asn Trp Ser Leu Ser Asp Lys Ser Glu Thr Ser Ala Ala Leu Met Pro
            260             265             270

Gly Ala Ser Pro Thr Gly Gly Trp Thr Asp Ser Asn Leu Ser Ala Ser
            275             280             285

Gly Lys Phe Val Arg Glu Gln Ile Arg
            290             295
```

```
<210>  2
<211>  297
<212>  PRT
<213>  Artificial Sequence

<220>
<223>  Ancestral endoglucanase catalytic domain

<400>  2
```

```
Thr Pro Val Glu Ile Asn Gly Gln Leu Gln Val Cys Gly Val Gln Leu
1               5               10              15

Cys Asn Gln Tyr Gly Lys Pro Ile Gln Leu Arg Gly Met Ser Thr His
            20              25              30

Gly Ile Gln Trp Phe Gly Asn Cys Val Asn Asn Ala Ser Leu Asp Ala
            35              40              45

Leu Ala Asn Asp Trp Arg Ala Asp Ile Phe Arg Ile Ala Met Tyr Ile
        50              55              60
```

```
Gln Glu Asp Gly Tyr Glu Thr Asn Pro Ala Gly Thr Asn Arg Val Asn
65                  70              75                  80

Asn Leu Val Glu Glu Ala Thr Ala Arg Gly Met Tyr Val Leu Ile Asp
                85              90                  95

Trp His Ile Leu Thr Pro Gly Asp Pro Asn Tyr Asn Leu Asp Arg Ala
            100             105             110

Lys Thr Phe Phe Ala Glu Ile Ala Ala Arg His Ala Ser Lys Thr Asn
            115             120             125

Val Ile Tyr Glu Ile Ala Asn Glu Pro Asn Gly Gly Val Ser Trp Ser
    130             135             140

Asn Thr Ile Lys Ser Tyr Ala Glu Glu Val Ile Pro Val Ile Arg Ala
145             150             155             160

Asn Asp Pro Asp Ser Val Val Ile Val Gly Thr Arg Gly Trp Ser Ser
            165             170             175

Asp Ser Thr Glu Ile Val Asn Asn Pro Val Asn Ala Ser Asn Ile Met
            180             185             190

Tyr Ala Phe His Phe Tyr Ala Ala Ser His Arg Asp Asn Tyr Arg Asp
            195             200             205

Glu Val Glu Arg Ala Ala Ala Arg Gly Leu Pro Val Phe Val Thr Glu
    210             215             220

Phe Gly Thr Val Thr Tyr Thr Gly Asp Gly Gly Asn Asp Leu Ala Ser
225             230             235             240

Ser Gln Lys Trp Leu Asp Leu Leu Asp Ala Arg Lys Ile Gly Trp Ala
            245             250             255

Asn Trp Asn Phe Ser Asp Lys Ala Glu Ser Ser Ala Ala Leu Arg Pro
            260             265             270

Gly Thr Cys Ala Gly Gly Ser Trp Thr Gly Thr Ser Leu Thr Pro Ser
            275             280             285

Gly Val Phe Val Arg Glu Arg Ile Arg
    290             295

<210>   3
<211>   297
<212>   PRT
```

<213> Artificial Sequence

<220>
<223> Ancestral endoglucanase catalytic domain

<400> 3

Thr Pro Val Glu Thr His Gly Gln Leu Ser Val Lys Gly Gly Gln Leu
1               5                   10                  15

Val Asp Glu Asn Gly Lys Pro Val Gln Leu Arg Gly Met Ser Ser His
                20                  25                  30

Gly Leu Gln Trp Phe Gly Asp Phe Val Asn Lys Asp Ser Met Lys Trp
        35                  40                  45

Leu Arg Asp Asp Trp Gly Ile Asn Val Phe Arg Val Ala Met Tyr Thr
        50                  55                  60

Ala Glu Asp Gly Tyr Ile Thr Asn Pro Ser Val Lys Asn Lys Val Lys
65                  70                  75                  80

Glu Ala Val Glu Ala Ala Ile Asp Leu Gly Met Tyr Val Ile Ile Asp
                85                  90                  95

Trp His Ile Leu Ser Asp Asn Asp Pro Asn Thr Tyr Lys Ala Gln Ala
                100                 105                 110

Lys Ala Phe Phe Gln Glu Met Ala Ala Leu Tyr Gly Asn Tyr Pro Asn
        115                 120                 125

Val Ile Tyr Glu Ile Ala Asn Glu Pro Asn Gly Asn Val Thr Trp Asn
        130                 135                 140

Asn Gln Ile Lys Pro Tyr Ala Glu Glu Val Ile Pro Val Ile Arg Ala
145                 150                 155                 160

Lys Asp Pro Asp Asn Ile Ile Ile Val Gly Thr Gly Thr Trp Ser Gln
                165                 170                 175

Asp Val His Asp Ala Ala Asp Asn Pro Leu Pro Asp Ser Asn Ile Met
                180                 185                 190

Tyr Ala Leu His Phe Tyr Ala Gly Thr His Gly Gln Phe Leu Arg Asp
        195                 200                 205

Arg Ile Asp Tyr Ala Leu Ser Lys Gly Ala Ala Ile Phe Val Thr Glu
        210                 215                 220

```
Trp Gly Thr Ser Asp Ala Ser Gly Asn Gly Gly Pro Phe Leu Pro Glu
225             230             235             240

Ser Gln Glu Trp Ile Asp Phe Met Asn Ser Arg Lys Ile Ser Trp Ala
            245             250             255

Asn Trp Ser Leu Ser Asp Lys Ser Glu Thr Ser Ala Ala Leu Met Pro
            260             265             270

Gly Ala Ser Pro Thr Gly Gly Trp Thr Glu Ser Gln Leu Ser Ala Ser
            275             280             285

Gly Lys Phe Val Arg Glu Gln Ile Arg
    290             295
```

```
<210>  4
<211>  150
<212>  PRT
<213>  Artificial Sequence

<220>
<223>  Carbohydrate binding domain

<400>  4
```

```
Gln Glu Asn Gly Ile Ser Val Gln Tyr Arg Ala Gly Asp Gly Ser Met
1               5               10              15

Asn Ser Asn Gln Ile Arg Pro Gln Leu Gln Ile Lys Asn Asn Gly Asn
            20              25              30

Thr Thr Val Asp Leu Lys Asp Val Thr Ala Arg Tyr Trp Tyr Asn Ala
            35              40              45

Lys Asn Lys Gly Gln Asn Val Asp Cys Asp Tyr Ala Gln Leu Gly Cys
    50              55              60

Gly Asn Val Thr Tyr Lys Phe Val Thr Leu His Lys Pro Lys Gln Gly
65              70              75              80

Ala Asp Thr Tyr Leu Glu Leu Gly Phe Lys Asn Gly Thr Leu Ala Pro
            85              90              95

Gly Ala Ser Thr Gly Asn Ile Gln Leu Arg Leu His Asn Asp Asp Trp
            100             105             110

Ser Asn Tyr Ala Gln Ser Gly Asp Tyr Ser Phe Phe Lys Ser Asn Thr
            115             120             125

Phe Lys Thr Thr Lys Lys Ile Thr Leu Tyr Asp Gln Gly Lys Leu Ile
```

24

130                    135                         140


Trp Gly Thr Glu Pro Asn
145                    150


<210>   5
<211>   15
<212>   PRT
<213>   Artificial Sequence

<220>
<223>   Linking domain

<400>   5

Gly Thr Lys Asp Ile Pro Glu Thr Pro Ala Lys Asp Lys Pro Thr
1                   5                   10                  15


<210>   6
<211>   87
<212>   PRT
<213>   Artificial Sequence

<220>
<223>   Carbohydrate binding domain

<400>   6

Phe Thr Val Gln Trp Lys Lys Ser Ser Ser Trp Glu Glu Gly Gly Lys
1                   5                   10                  15


Lys Cys Gly Gly Tyr Glu Ile Val Ile Thr Asn Asn Gly Asp Thr Val
            20                  25                  30


Asn Ser Trp Thr Ala Lys Val Thr Val Pro Gly Asn Thr Lys Leu Met
            35                  40                  45


Ser Gln Trp Asn Gly Ile Phe Ser Ile Ser Gly Asn Thr Met Thr Val
            50                  55                  60


Lys Asn Glu Ser Tyr Asn Gly Thr Ile Glu Lys Gly Lys Ser Ile Ser
65                  70                  75                  80


Phe Gly Phe Asn Tyr Ser Ala
                    85


<210>   7
<211>   87
<212>   PRT
<213>   Artificial Sequence

<220>
<223>   Carbohydrate binding domain

<400> 7

Tyr Gln Leu Lys Val Thr Val Ser Ser Ser Trp Glu Ser Gly Asp Gly
1               5               10              15

Tyr Gly Gly Thr Tyr Ser Leu Ser Phe Thr Asn Arg Ser Ala Ala Val
            20              25              30

Lys Ala Trp Asp Ala Gln Ile Glu Val Pro Glu Gly Ser Lys Val Thr
        35              40              45

Glu Ala Trp Gly Cys Glu Thr Gly Ile Asp Gly Thr Ile Leu Thr Val
        50              55              60

Thr Ala Lys Asp Trp Gly Ala Ala Val Ala Lys Gly Ser Thr Val Glu
65              70              75              80

Ile Gly Phe Asn Met Asp Thr
                85

<210>  8
<211>  84
<212>  PRT
<213>  Artificial Sequence

<220>
<223>  Carbohydrate binding domain

<400>  8

Tyr Lys Ala Asp Phe Lys Val Val Asn Ser Trp Glu Glu Gly Gly Lys
1               5               10              15

Lys Cys Tyr Gln Leu Ser Gly Thr Leu Thr Asn Leu Ser Ser Ser Ile
            20              25              30

Ser Ser Trp Thr Val Val Phe Asp Ala Gly Asn Gly Ala Glu Ile Lys
        35              40              45

Gln Phe Trp Asn Ser Lys Cys Thr Ile Ser Gly Asn Lys Ile Thr Val
        50              55              60

Gly Pro Ala Asp Tyr Asn Ser Arg Ile Gly Thr Gly Ala Ser Val Ser
65              70              75              80

Asp Val Gly Met

<210>  9
<211>  87
<212>  PRT

26

<210> Artificial Sequence

<220>
<223> Carbohydrate binding domain

<400> 9

Phe Tyr Ala Glu Ile Gly His Asp Ser Thr Trp Glu Ala Ser Gly Lys
1               5                   10                  15

Thr Cys Ala Thr Glu Asn Ile Asn Ile Tyr Asn Lys Thr Ser Ser Val
            20                  25                  30

Ser Gly Trp Lys Leu Asp Val Ile Tyr Lys Gly Lys Pro Ala Ile Glu
        35                  40                  45

Asp Ile Trp Asn Gly Glu Lys Lys Ile Asn Glu Tyr Thr Val Ser Ile
        50                  55                  60

Thr Pro Ala Asp Tyr Asn Gln Asp Ile Pro Ala Gly Gly Ser Val Asn
65                  70                  75                  80

Val Gly Tyr Asn Ile Ala Ser
                85

<210> 10
<211> 87
<212> PRT
<213> Artificial Sequence

<220>
<223> Carbohydrate binding domain

<400> 10

Tyr Tyr Ala Ser Ile Ser Asp Asn Ser Ser Trp Gln Glu Asn Gly Lys
1               5                   10                  15

Asn Ala Ala Thr Lys Asn Val Ile Ile Tyr Asn Lys Asp Lys Lys Val
            20                  25                  30

Thr Gly Trp Lys Ile Glu Leu Val Phe Ala Ser Glu Pro Glu Leu Ala
        35                  40                  45

Asp Ile Trp Gly Gly Lys Ala Glu Val Asn Gly Asp Thr Ile Thr Val
        50                  55                  60

Val Gly Tyr Asp Tyr Thr Ala Glu Leu Lys Ala Gly Gly Asn Val Asn
65                  70                  75                  80

Phe Gly Phe Asn Val Lys Ala
                85

<210> 11
<211> 77
<212> PRT
<213> Artificial Sequence

<220>
<223> Carbohydrate binding domain

<400> 11

Trp Ala Glu Gln Asn Gln Thr Ala Phe Gln Tyr Glu Leu Leu Phe Ser
1               5                   10                  15

Asn Gln Ser Glu Ala Phe Gly Thr Trp Lys Val Ile Leu Asp Thr Gly
            20                  25                  30

Lys Glu Val Lys Val Gln Asn Ser Trp Asn Cys Ser Leu Glu Ala Asp
        35                  40                  45

Gly Asn Leu Leu Thr Phe Thr Pro Ala Asp Tyr Asn Ala Lys Leu Ala
    50                  55                  60

Lys Gly Ala Glu Met Ala Asp Val Gly Met Ile Leu Val
65                  70                  75

<210> 12
<211> 85
<212> PRT
<213> Artificial Sequence

<220>
<223> Carbohydrate binding domain

<400> 12

Met Gln Phe Lys Gln Ser Asn Ser Trp Glu Gly Asn Gly Arg Phe Tyr
1               5                   10                  15

Ala Gln Tyr Asp Leu Ile Ile Asp Asn Lys Glu Asp Val Ile Ser Asp
            20                  25                  30

Trp Thr Phe Lys Ile Asn Thr Thr Asn Gly Thr Thr Leu Glu Gln Ser
        35                  40                  45

Trp Asn Cys Thr Val Asp Thr Ser Asp Leu Gln Trp Ser Val Val Pro
        50                  55                  60

Val Asp Tyr Asn Lys Ile Ile Glu Ser Gln Ala Gln Met Asn Asn Val
65                  70                  75                  80

Gly Phe Ile Ile Ser

85

```
<210>    13
<211>    147
<212>    PRT
<213>    Artificial Sequence

<220>
<223>    Carbohydrate binding domain

<400>    13

Asn Gly Ile Ser Val Gln Tyr Arg Ala Gly Asp Gly Ser Met Asn Ser
1               5                   10                  15


Asn Gln Ile Arg Pro Gln Leu Gln Ile Lys Asn Asn Gly Asn Thr Thr
            20                  25                  30


Val Asp Leu Lys Asp Val Thr Ala Arg Tyr Trp Tyr Asn Ala Lys Asn
        35                  40                  45


Lys Gly Gln Asn Val Asp Cys Asp Tyr Ala Gln Leu Gly Cys Gly Asn
    50                  55                  60


Val Thr Tyr Lys Phe Val Thr Leu His Lys Pro Lys Gln Gly Ala Asp
65                  70                  75                  80


Thr Tyr Leu Glu Leu Gly Phe Lys Asn Gly Thr Leu Ala Pro Gly Ala
                85                  90                  95


Ser Thr Gly Asn Ile Gln Leu Arg Leu His Asn Asp Asp Trp Ser Asn
                100                 105                 110


Tyr Ala Gln Ser Gly Asp Tyr Ser Phe Phe Lys Ser Asn Thr Phe Lys
            115                 120                 125


Thr Thr Lys Lys Ile Thr Leu Tyr Asp Gln Gly Lys Leu Ile Trp Gly
        130                 135                 140


Thr Glu Pro
145


<210>    14
<211>    137
<212>    PRT
<213>    Artificial Sequence

<220>
<223>    Carbohydrate binding domain

<400>    14
```

EP 3 872 173 A1

```
Asn Gly Ile Ser Val Gln Tyr Arg Ala Gly Asp Gly Ser Met Asn Ser
1               5                   10                  15

Asn Gln Ile Arg Pro Gln Leu Gln Ile Lys Asn Asn Gly Asn Thr Thr
            20                  25                  30

Val Asp Leu Lys Asp Val Thr Ala Arg Tyr Trp Tyr Lys Ala Lys Asn
            35                  40                  45

Lys Gly Gln Asn Val Asp Cys Asp Tyr Ala Gln Ile Gly Cys Gly Asn
        50                  55                  60

Val Thr Tyr Lys Phe Val Thr Leu His Lys Pro Lys Gln Gly Ala Asp
65                  70                  75                  80

Thr Tyr Leu Glu Leu Gly Phe Lys Asn Gly Thr Leu Ala Pro Gly Ala
                85                  90                  95

Ser Thr Gly Asn Ile Gln Leu Arg Leu His Asn Asp Asp Trp Ser Asn
            100                 105                 110

Tyr Ala Gln Ser Gly Asp Tyr Ser Phe Phe Lys Ser Asn Thr Phe Lys
        115                 120                 125

Thr Thr Lys Lys Ile Thr Leu Tyr Asp
    130                 135

<210>   15
<211>   147
<212>   PRT
<213>   Artificial Sequence

<220>
<223>   Carbohydrate binding domain

<400>   15

Asn Gly Ile Ser Val Gln Tyr Lys Ala Gly Asp Gly Gly Val Asn Ser
1               5                   10                  15

Asn Gln Ile Arg Pro Gln Leu His Ile Lys Asn Asn Gly Asn Ala Thr
            20                  25                  30

Val Asp Leu Lys Asp Val Thr Ala Arg Tyr Trp Tyr Asn Ala Lys Asn
            35                  40                  45

Lys Gly Gln Asn Phe Asp Cys Asp Tyr Ala Gln Ile Gly Cys Gly Asn
        50                  55                  60

Leu Thr His Lys Phe Val Thr Leu His Lys Pro Lys Gln Gly Ala Asp
```

30

```
            65                      70                      75                      80


            Thr Tyr Leu Glu Leu Gly Phe Lys Thr Gly Thr Leu Ser Pro Gly Ala
                        85                      90                      95


            Ser Thr Gly Asn Ile Gln Leu Arg Leu His Asn Asp Asp Trp Ser Asn
                        100                     105                     110


            Tyr Ala Gln Ser Asp Asp Tyr Ser Phe Phe Gln Ser Asn Thr Phe Lys
                        115                     120                     125


            Thr Thr Lys Lys Ile Thr Leu Tyr His Gln Gly Lys Leu Ile Trp Gly
                        130                     135                     140


            Thr Glu Pro
            145


            <210>  16
            <211>  63
            <212>  PRT
            <213>  Artificial Sequence

            <220>
            <223>  Carbohydrate binding domain

            <400>  16

            Pro Gly Glu Pro Glu Pro Asp Pro Gly Glu Pro Asp Pro Thr Pro Pro
            1               5                       10                      15


            Ser Asp Gly Asp Tyr Pro Ala Trp Asp Pro Asn Thr Ile Tyr Thr Asp
                        20                      25                      30


            Glu Ile Val Tyr His Asn Gly Gln Leu Trp Gln Ala Lys Trp Trp Thr
                        35                      40                      45


            Gln Asn Gln Glu Pro Gly Asp Tyr Gly Pro Trp Glu Pro Leu Asn
                50                      55                      60


            <210>  17
            <211>  145
            <212>  PRT
            <213>  Artificial Sequence

            <220>
            <223>  Carbohydrate binding domain

            <400>  17

            Gly Asn Leu Val Val Gln Tyr Lys Val Gly Asp Thr Ser Ala Thr Asp
            1               5                       10                      15
```

```
Asn Gln Met Lys Pro Ser Phe Asn Ile Lys Asn Asn Gly Thr Thr Pro
            20              25              30


Val Asn Leu Ser Gly Leu Lys Leu Arg Tyr Tyr Phe Thr Lys Asp Gly
            35              40              45


Thr Asp Met Ser Ala Ser Ile Asp Trp Ala Gln Ile Gly Ala Ser Asn
            50              55              60


Ile Ser Ala Ala Phe Ala Asp Phe Thr Gly Ser Asn Thr Asp Thr Tyr
65              70              75              80


Val Glu Leu Ser Phe Ser Ala Gly Ser Ile Pro Ala Gly Gly Gln Thr
            85              90              95


Gly Asp Ile Gln Leu Arg Met Tyr Lys Thr Asp Trp Ser Asn Phe Asn
            100             105             110


Glu Ala Asn Asp Tyr Ser Tyr Asp Gly Ala Lys Thr Tyr Ala Asp Trp
            115             120             125


Asn Arg Val Thr Leu His Gln Asn Gly Thr Leu Val Trp Gly Thr Thr
            130             135             140


Pro
145



<210> 18
<211> 57
<212> PRT
<213> Artificial Sequence

<220>
<223> Carbohydrate binding domain

<400> 18

Asp Pro Gly Glu Pro Asp Pro Thr Pro Pro Ser Asp Gly Glu Tyr Pro
1               5               10              15


Ala Trp Asp Pro Asn Gln Ile Tyr Thr Asn Glu Ile Val Tyr His Asn
            20              25              30


Gly Gln Leu Trp Gln Ala Lys Trp Trp Thr Gln Asn Gln Glu Pro Gly
            35              40              45


Asp Tyr Gly Pro Trp Glu Pro Leu Asn
            50              55


<210> 19
```

<211> 144
<212> PRT
<213> Artificial Sequence

<220>
<223> Carbohydrate binding domain

<400> 19

Gly Ser Leu Val Leu Gln Tyr Arg Ala Ala Asp Thr Asn Ala Gly Asp
1               5                   10                  15

Asn Ala Ile Lys Pro His Phe Asn Ile Arg Asn Thr Gly Ala Ser Pro
            20                  25                  30

Val Asp Leu Ser Gly Val Lys Leu Arg Tyr Tyr Phe Thr Lys Asp Gly
        35                  40                  45

Ile Pro Leu Ser Phe Ala Val Asp Trp Ala Gln Val Gly Ser Pro Asn
    50                  55                  60

Val Lys Gly Thr Phe Gly Ser Ala Ser Gly Ala Gly Ala Asp Thr Tyr
65                  70                  75                  80

Leu Glu Val Ser Phe Thr Gly Ser Ile Pro Ala Gly Gly Gln Thr Gly
                85                  90                  95

Glu Ile Gln Thr Arg Ile His Lys Ser Asp Trp Ser Ser Phe Gln Glu
            100                 105                 110

Ser Gly Asp Tyr Ser Tyr Asp Pro Gly Lys Thr Tyr Ala Asp Trp Ser
            115                 120                 125

Lys Val Thr Leu Tyr Arg Asp Gly Thr Arg Val Trp Gly Val Glu Pro
        130                 135                 140

<210> 20
<211> 147
<212> PRT
<213> Artificial Sequence

<220>
<223> Carbohydrate binding domain

<400> 20

Asn Ile Thr Ser Ala Val Tyr Thr Ile Ser Asn Thr Asp Pro Val Lys
1               5                   10                  15

Gln Val Ser Ala Pro Thr Phe Ser Leu Gln Ser Ala Gln Thr Val Thr
            20                  25                  30

33

Leu Thr Ser Ser Asp Asn Asp Ser Val Ile His Tyr Thr Thr Asp Gly
        35                  40              45

Thr Pro Thr Ser Ser Ser Pro Val Tyr Thr Asn Met Thr Asp Ser Ser
        50                  55              60

Val Val Thr Asn Thr Tyr Thr Ile Thr Asn Glu Thr Ser Asn Ser Pro
65                  70              75                  80

Val Glu Val Glu Val Glu Tyr Lys Thr Thr Val Lys Val Thr Leu Thr
                85              90                  95

Asn Asn Ser Ser Thr Pro Ile Asn Gly Trp Lys Leu Ser Trp Ile Asn
            100             105             110

Asn Met Trp Thr Ala Asn Tyr Ser Ile Lys Asp Ser Ala Ile Val Lys
            115             120             125

Asp Tyr Asn Asn Ile Ile Pro Ala Asn Gly Gly Thr Gln Ile Phe Gly
        130             135             140

Phe Ser Ile
145


<210>   21
<211>   147
<212>   PRT
<213>   Artificial Sequence

<220>
<223>   Carbohydrate binding domain

<400>   21

Gln Gly Ile Val Leu Gln Tyr Arg Thr Gly Asp Thr Asn Thr Lys Asp
1               5               10                  15

Asn Ala Ile Arg Pro Glu Phe Asn Ile Lys Asn Thr Gly Asn Thr Ala
            20              25              30

Val Lys Leu Ser Asp Leu Lys Ile Arg Tyr Tyr Tyr Thr Asp Glu Ser
        35                  40                  45

Lys Gly Gln Gln Leu Phe Val Asp Trp Ala Lys Val Gly Asn Glu Lys
        50                  55                  60

Val Lys Ala Thr Phe Val Ala Leu Pro Glu Pro Lys Ala Lys Ala Asp
65                  70                  75                  80

Lys Tyr Val Glu Ile Ser Phe Thr Asp Gly Thr Ile Gln Pro Gly Gly

34

```
                      85                      90                      95

        Glu Ser Gly Glu Ile Gln Pro Arg Ile His Ala Ala Asn Trp Ser Asn
                    100                 105                 110


        Phe Asp Glu Thr Asn Asp Tyr Ser Tyr Gly Ala Ser Gln Thr Phe Ala
                    115                 120                 125


        Asn Trp Asp His Ala Thr Val Tyr Gln Gln Gly Lys Leu Val Trp Gly
                    130                 135                 140


        Ile Glu Pro
        145


        <210>   22
        <211>   147
        <212>   PRT
        <213>   Artificial Sequence

        <220>
        <223>   Carbohydrate binding domain

        <400>   22

        Glu Gly Ile Val Leu Gln Tyr Arg Thr Asp Asp Thr Asn Ala Lys Asp
        1               5                   10                  15


        Asn Ala Ile Arg Pro Gln Phe Asn Ile Lys Asn Thr Gly Lys Thr Ala
                    20                  25                  30


        Val Lys Leu Ser Asp Leu Lys Ile Arg Tyr Tyr Tyr Thr Asp Glu Ser
                    35                  40                  45


        Lys Ala Gln Gln Phe Phe Val Asp Trp Ala Lys Ile Gly Asn Glu Lys
                    50                  55                  60


        Val Lys Ala Thr Phe Val Thr Leu Pro Asn Pro Lys Ser Lys Ala Asp
        65                  70                  75                  80


        Lys Tyr Val Glu Ile Ser Phe Thr Asp Gly Thr Ile Gln Pro Gly Gly
                        85                  90                  95


        Glu Thr Gly Glu Ile Gln Ser Arg Ile His Ala Ala Asn Trp Ser Asn
                    100                 105                 110


        Phe Asp Glu Thr Asn Asp Tyr Ser Tyr Gly Ala Ala Gln Thr Phe Ala
                    115                 120                 125


        Asp Trp Asp His Gly Thr Val Tyr Gln Gln Gly Lys Leu Val Trp Gly
                    130                 135                 140
```

Ile Glu Pro
145

<210> 23
<211> 107
<212> PRT
<213> Artificial Sequence

<220>
<223> Carbohydrate binding domain

<400> 23

Gln Ala Ala Gly Leu Thr Ala Thr Val Thr Lys Glu Ser Ser Trp Asp
1               5                   10                  15

Asn Gly Tyr Ser Ala Ser Val Thr Val Arg Asn Asp Thr Ser Ser Val
            20                  25                  30

Ser Gln Trp Glu Val Val Leu Thr Leu Pro Gly Gly Thr Thr Val Ala
        35                  40                  45

Gln Val Trp Asn Ala Gln His Thr Ser Ser Gly Asn Ser His Thr Phe
    50                  55                  60

Thr Gly Val Ser Trp Asn Ser Thr Ile Pro Pro Gly Gly Thr Ala Ser
65                  70                  75                  80

Phe Gly Phe Ile Ala Ser Gly Ser Gly Glu Pro Thr His Cys Thr Ile
                85                  90                  95

Asn Gly Ala Pro Cys Asp Glu Gly Ser Glu Pro
            100                 105

<210> 24
<211> 106
<212> PRT
<213> Artificial Sequence

<220>
<223> Carbohydrate binding domain

<400> 24

Ala Ala Thr Gly Cys Ser Val Thr Tyr Thr Thr Asn Ser Trp Ser Asn
1               5                   10                  15

Gly Phe Thr Ala Asn Val Thr Val Thr Asn Leu Gly Asp Ala Ile Gly
            20                  25                  30

Asn Trp Thr Leu Gly Phe Ser Phe Pro Ser Gly Gln Arg Val Thr Gln

                    35                          40                          45

        Gly Trp Ser Ala Ile Trp Ser Gln Ser Gly Asn Ala Val Thr Ala Arg
            50                  55                  60

        Ser Glu Ser Trp Asn Gly Asn Leu Ala Thr Gly Ala Ser Thr Ser Ile
        65                  70                  75                  80

        Gly Phe Asn Gly Ser Phe Thr Gly Ser Pro Thr Ser Phe Thr Leu Asn
                        85                  90                  95

        Gly Val Pro Cys Thr Gly Ser Thr Thr Thr
                    100                 105

        <210>   25
        <211>   103
        <212>   PRT
        <213>   Artificial Sequence

        <220>
        <223>   Carbohydrate binding domain

        <400>   25

        Glu Ala Ala Ser Phe Thr Val Thr Asn Ser Trp Ser Thr Gly Tyr Gln
        1               5                   10                  15

        Gly Glu Val Thr Val Ser Asn Pro Ala Ser Ser Ile Asn Thr Trp Lys
                    20                  25                  30

        Val Gln Leu Thr Leu Pro Ala Gly Ser Thr Ile Gly Gln Ala Trp Asn
                    35                  40                  45

        Ala Thr Leu Ala Thr Gly Gly Gln Thr Phe Thr Phe Thr Pro Ala Gly
            50                  55                  60

        Trp Asn Gly Thr Ile Ala Gly Gly Ser Ala Thr Ser Phe Gly Phe Val
        65                  70                  75                  80

        Val Thr Gly Thr Gly Arg Pro Thr Ser Cys Thr Val Asn Gly Gln Ala
                        85                  90                  95

        Cys Thr Gly Leu Thr Gly Ala
                    100

        <210>   26
        <211>   107
        <212>   PRT
        <213>   Artificial Sequence

        <220>

<223>    Carbohydrate binding domain

<400>    26

Ala Ala Thr Gly Cys His Val Asp Tyr Thr Val Thr Asn Gln Trp Gln
1               5                   10                  15

Gly Gly Phe Gln Ala Ala Val Lys Val Thr Asn Leu Gly Asp Ala Ile
            20                  25                  30

Thr Gly Trp Leu Leu Arg Phe Thr Phe Pro Ala Gly Gln Lys Val Ala
            35                  40                  45

Gln Gly Trp Asn Ala Thr Trp Ala Gln Ser Gly Ala Thr Ala Thr Ala
            50                  55                  60

Ala Asn Ala Asp Trp Asn Arg Thr Leu Thr Thr Gly Ala Thr Thr Glu
65                  70                  75                  80

Leu Gly Phe Thr Gly Thr Thr Thr Gly Val Pro Ala Ser Phe Thr Leu
            85                  90                  95

Asn Gly Val Thr Cys Thr Gly Ser Thr Thr Thr
            100                 105

<210>    27
<211>    106
<212>    PRT
<213>    Artificial Sequence

<220>
<223>    Carbohydrate binding domain

<400>    27

Ala Ala Thr Gly Cys Ala Val Thr Tyr Thr Thr Asn Ser Trp Gln Gly
1               5                   10                  15

Gly Phe Thr Ala Thr Val Ala Val Arg Asn Leu Gly Asp Pro Val Ser
            20                  25                  30

Asn Trp Thr Leu Gly Phe Thr Phe Pro Gly Gly Gln Arg Val Val Gln
            35                  40                  45

Gly Trp Ser Ala Thr Trp Gln Gln Ser Gly Ser Ala Val Thr Ala Arg
            50                  55                  60

Ser Leu Asp Tyr Asn Gly Ala Leu Gly Thr Gly Ala Ser Thr Thr Ile
65                  70                  75                  80

Gly Phe Asn Gly Ser Trp Thr Gly Ser Pro Thr Ser Phe Thr Leu Asn

38

85                    90                    95

Gly Thr Val Cys Thr Gly Gly Thr Ser Thr
            100                   105

<210> 28
<211> 106
<212> PRT
<213> Artificial Sequence

<220>
<223> Carbohydrate binding domain

<400> 28

Ala Ala Thr Gly Cys Ala Val Thr Tyr Thr Thr Asn Ser Trp Gln Gly
1               5                   10                  15

Gly Phe Thr Ala Thr Val Ala Val Arg Asn Leu Gly Asp Pro Val Ser
            20                  25                  30

Asn Trp Thr Leu Gly Phe Thr Phe Pro Gly Gly Gln Arg Val Val Gln
            35                  40                  45

Gly Trp Ser Ala Thr Trp Gln Gln Ser Gly Ser Ala Val Thr Ala Arg
        50                  55                  60

Ser Leu Asp Tyr Asn Gly Ala Leu Gly Thr Gly Ala Ser Thr Thr Ile
65                  70                  75                  80

Gly Phe Asn Gly Ser Trp Thr Gly Ser Pro Thr Ser Phe Thr Leu Asn
            85                  90                  95

Gly Thr Val Cys Thr Gly Gly Thr Ser Thr
            100                   105

<210> 29
<211> 100
<212> PRT
<213> Artificial Sequence

<220>
<223> Carbohydrate binding domain

<400> 29

Glu Ser Glu Asn Ala Thr Ile Ser Arg Gly Val Val Glu Ala Asn His
1               5                   10                  15

Thr Gly Phe Thr Gly Ser Gly Phe Val Asn Tyr Asp Asn Val Thr Gly
            20                  25                  30

```
Ser Tyr Val Glu Tyr Thr Val Asn Ala Ala Gln Ala Gly Pro His Thr
        35              40              45

Leu Thr Phe Arg Tyr Ala Asn Gly Thr Thr Ala Asn Arg Pro Leu Asp
        50              55              60

Ile Thr Val Asn Gly Ser Ile Ala Val Asp Asp Leu Gly Phe Ala Gly
65              70              75              80

Thr Gly Ala Trp Ser Thr Val Thr Thr Thr Val Asn Leu Ala Ala Gly
                85              90              95

Ser Asn Lys Ile
            100


<210>   30
<211>   107
<212>   PRT
<213>   Artificial Sequence

<220>
<223>   Carbohydrate binding domain

<400>   30

Asn Ala Ala Gly Cys Arg Val Asp Tyr Thr Val Thr Asn Gln Trp Gln
1               5               10              15

Gly Gly Phe Gln Ala Gly Val Lys Ile Thr Asn Leu Gly Asp Thr Val
                20              25              30

Arg Gly Trp Thr Leu Lys Phe Thr Leu Pro Thr Gly Gln Lys Val Val
            35              40              45

Gln Gly Trp Ser Ala Ala Trp Ser Gln Ser Gly Ser Thr Val Thr Val
        50              55              60

Ala Gly Ala Asp Trp Asn Gly Thr Leu Ala Thr Gly Ala Ser Ala Asp
65              70              75              80

Thr Gly Phe Val Gly Ser Phe Thr Gly Lys Pro Thr Ala Phe Thr Leu
                85              90              95

Asn Gly Val Ala Cys Thr Gly Ser Val Asp Asp
            100             105


<210>   31
<211>   105
<212>   PRT
<213>   Artificial Sequence
```

<220>
<223>    Carbohydrate binding domain

<400>    31

Ala Ala Thr Gly Cys Lys Ala Glu Tyr Thr Ile Thr Ser Gln Trp Glu
1                5                   10                  15

Gly Gly Phe Gln Ala Gly Val Lys Ile Thr Asn Leu Gly Asp Pro Val
                20                  25                  30

Ser Gly Trp Thr Leu Gly Phe Thr Met Pro Ala Gly Gln Arg Leu Val
                35                  40                  45

Gln Gly Trp Asn Ala Thr Trp Ser Gln Ser Gly Ser Ala Val Thr Ala
            50                  55                  60

Gly Gly Val Asp Trp Asn Arg Thr Leu Ala Thr Gly Ala Ser Ala Asp
65                  70                  75                  80

Leu Gly Phe Val Gly Ser Phe Thr Gly Ala Pro Thr Ser Phe Thr Leu
                85                  90                  95

Asn Gly Ala Thr Cys Ser Gly Ser Val
                100                 105

<210>    32
<211>    81
<212>    PRT
<213>    Artificial Sequence

<220>
<223>    Carbohydrate binding domain

<400>    32

Asp Gly Ile Gln Thr Glu Ser Thr Thr Asp Thr Gly Gly Gly Leu Asn
1                5                   10                  15

Ile Gly Trp Thr Asp Ala Gly Asp Trp Thr Ser Pro Ala Ala Gly Arg
                20                  25                  30

Tyr Lys Val Ser Tyr Arg Asn Ser Gly Met Leu Gln Leu Glu Ala Ala
                35                  40                  45

Gly Gly Phe Pro Thr Tyr Gly Ser Ile Thr Gly Gly Trp Gln Ser Trp
            50                  55                  60

Gln Thr Ile Ser His Glu Val Asn Leu Asn Trp Ile Lys Val Glu Pro
65                  70                  75                  80

Ala

<210> 33
<211> 81
<212> PRT
<213> Artificial Sequence

<220>
<223> Carbohydrate binding domain

<400> 33

```
Pro Thr Glu Pro Thr Asn Pro Gly Asn Gly Thr Thr Gly Asp Val Val
1               5                   10                  15

Leu Gln Tyr Arg Asn Val Asp Asn Asn Pro Ser Asp Asp Ala Ile Arg
            20                  25                  30

Met Ala Val Asn Ile Lys Asn Thr Gly Ser Thr Pro Ile Lys Leu Ser
            35                  40                  45

Asp Leu Gln Val Arg Tyr Tyr Phe His Asp Asp Gly Lys Pro Gly Ala
        50                  55                  60

Asn Leu Phe Val Asp Trp Ala Asn Val Gly Pro Asn Asn Ile Val Thr
65                  70                  75                  80
```

Ser

<210> 34
<211> 81
<212> PRT
<213> Artificial Sequence

<220>
<223> Carbohydrate binding domain

<400> 34

```
Pro Thr Glu Pro Thr Asn Pro Gly Asn Gly Thr Thr Gly Asp Ile Val
1               5                   10                  15

Leu Gln Tyr Arg Asn Val Asp Asn Asn Pro Ser Asp Asp Ala Ile Arg
            20                  25                  30

Met Ala Phe Asn Ile Lys Asn Thr Gly Ser Thr Pro Ile Lys Leu Ser
            35                  40                  45

Asp Leu Gln Val Arg Tyr Tyr Phe His Asp Asp Gly Lys Pro Gly Ala
        50                  55                  60
```

Asn Leu Phe Val Asp Trp Ala Asn Val Gly Pro Asn Asn Ile Val Thr
65                    70                    75                    80

Ser